# EUROPÄISCHE PATENTSCHRIFT

(11) **EP 0 821 237 B1**
(45) Veröffentlichungstag und Bekanntmachung des Hinweises auf die Patenterteilung: **03.03.2004**
(21) Anmeldenummer: 97112255.1
(22) Anmeldetag: 17.07.1997
(51) Int. Cl.: G01N 33/569, C07K 14/20, C07K 16/12

(54) **Immunassay zum Nachweis von anti-B. burgdorferi Antikörpern und Verfahren zur Serodiagnose bei Lyme Borreliose, diagnostische Mittel und Testkits zur Durchführung der Verfahren**
Immunoassay for detection of anti-B. burgdorferi antibodies and method for serological detection of Lyme borreliosis, diagnostic agent and test kits for carrying out the methods
Essai immunologique pour la détection des anticorps anti-B. burgdorferi et méthode serodiagnostic pour Lyme borreliose, agent diagnostique et kits de tests pour la mise en oeuvre des procédés

(30) Priorität: 22.07.1996 DE 19629543
(43) Veröffentlichungstag der Anmeldung: 28.01.1998
(73) Patentinhaber: Baxter Healthcare S.A., 8304 Wallisellen (CH)
(72) Erfinder: Crowe, Brian, Dr., 1210 Wien (AT); Lauchart, Manfred, 1050 Wien (AT); Dorner, Friedrich, Prof., 1230 Wien (AT)
(74) Vertreter: Polz, Leo, Dr.

(56) Entgegenhaltungen:
- WO-A-93/04175
- WO-A-94/25596
- WO-A-95/14781

## Beschreibung

Die Erfindung betrifft einen Immunassay zum Nachweis von anti-*B*. *burgdorferi* Antikörpern in einer Probe. Im speziellen betrifft die Erfindung diagnostische Mittel, enthaltend als Reagenskomponenten diskriminierende Antigene abgeleitet von *B. afzelii* und/oder OspC Antigen von ausgewählten RFLP-Typen von Borrelia burgdorferi s.l. sowie ein Verfahren zur Serodiagnose von Lyme Borreliose mit einer verbesserten Spezifität und Sensitivität und zur differentiellen Detektion von Antikörpern gegen verschiedene Serotypen von *B*. *burgdorferi*.

Lyme Borreliose (LB) ist eine der häufigsten der von Zecken übertragenen Infektionskrankheiten des Menschen. Sie ist eine multisystemische Erkrankung, die durch die Spirochäte *Borrelia burgdorferi* hervorgerufen wird. *B. burgdorferi* gehört zur Familie der Spirochäten, i.e. schraubenförmige Bakterien von 8-30 µm Länge, die aus einer äusseren Hülle, den Endoflaggellen im Periplasma und dem Protoplastenzylinder bestehen. Zu den human-pathogenen Vertretern der Spirochäten gehören neben *B. burgdorferi* die Rückfallfieberborrelien (z.B. *B. recurrentis*), der Erreger der Syphilis (*Treponema* z. B. *T. palladium*) und die Leptospiren. Zu *B. burgdorferi sensu lato* gehören die Vertreter der Spezies *B. burgdorferi sensu stricto, B. garinii, B. afzelii, B. japonica* und *B. andersonii*, wobei alle drei erstgenannten Vertreter in Europa weit verbreitet sind, während *B. burgdorferi sensu stricto* vor allem in Nordamerika vorherrschend ist.

Die nahe immunologische Verwandtschaft der Erreger erschwert den serologischen Nachweis von Antikörpern bei Syphilis und Lyme Borreliose mit den bisher verfügbaren Tests.

Das klinische Spektrum der Lyme Borreliose umfasst drei Syndrome (EM, ACA und MPN-GBB), die lange Zeit als eigenständige Krankheitsbilder beschrieben wurden. Die Lyme Borreliose wird, ähnlich wie bei der Syphilis, in drei verschiedene Stadien eingeteilt, die in einem unterschiedlichen Zeitraum nach Infektion mit dem Erreger auftreten:
Stadium I (Tage bis Wochen): Erythema migrans (EM), Lymphadenosis cutis benigna (LCB)
Stadium II (Wochen bis Monate): sekundäre EM, LCB, Meningitis, Enzephalitis, Myelitis, Arthralgien
Stadium III (Monate bis Jahre): ACA, Enzephalomyelitis, Arthritis.

Lyme Borreliose kann durch Verabreichung von Antibiotika, wie Penicillin G, Tetracyclinen, Erythromycin oder Cephalosporinen, therapiert werden. In einigen Fällen heilt die Lyme Borreliose in den frühen Stadien oft spontan aus,jedoch sind auch Spätmanifestationen nicht ausgeschlossen. Zudem ist eine klinische Heilung nach Antibiotikatherapie bei Spätmanifestation nur in wenigen Fälle zu erzielen. Lyme Borreliose sollte daher möglichst frühzeitig diagnostiziert werden.

Bei der Vielfalt von Symptomen der Lyme Borreliose ist ein Infektionsnachweis durch klinische Diagnose in Verbindung mit serologischen Methoden unumgänglich.

Bei einer Lyme Borreliose werden jedoch nicht immer alle drei Stadien durchlaufen; fliessende Übergänge und symptomfreie Intervalle sind ebenfalls möglich. Da auch Stadien übersprungen werden, kann sich die Erkrankung in jeder der drei Phasen erstmals manifestieren. Dieser Umstand führt aber immer wieder zu Fehlbeurteilungen, da oftmals angenommen wird, dass die typische Manifestation der Lyme Borreliose, nämlich das EM durchlaufen werden muss, bevor eine spätere Manifestation auftreten kann. Da das vielfältige Erscheinungsbild der Lyme Borreliose oft auch anderen Krankheitsformen ähnelt, kann es bei rein klinischen Diagnosen zu Fehlbeurteilungen kommen. Diese Gefahr erhöht sich um so mehr, wenn die Anamnese des Patienten keinen Anhaltspunkt auf einen vorangegangenen Zeckenbiss aufweist.

Der Antikörpernachweis (IgM, IgG) wird im Serum und bei neurologischen Manifestationen auch aus dem Liquor durchgeführt. Serologische Untersuchungen zeigten bei Patienten mit EM überwiegend positive IgM-Befunde, bei neurologischen Manifestationen überwiegend positive IgG-Befunde. Bei den Spätmanifestationen ACA und Lyme-Arthritis ist der IgG-Titer regelmässig erhöht und IgM nur in Ausnahmefällen nachweisbar (Wilske et al. 1984. Infection 12:331-337, Herzer et al. 1986. Zbl. Bakt.Hyg. A 263:268-274). Exponierte Personen, wie Waldarbeiter, weisen mit zunehmendem Alter oft einen signifikant erhöhten Antikörperspiegel auf.

Der serologische Befund ist abhängig vom Stadium der Erkrankung, der Dauer der Symptome und einer eventuell schon erfolgten Antibiotika-Therapie. So ist der Antikörpernachweis mit bisher verfügbaren Testsystemen bei EM nur in 20-50%, bei neurologischen Manifestationen in 50-90% und bei ACA und Arthritis in 90-100% der Fälle möglich. Erhöhte Antikörper-Titer sind diagnostisch nicht immer eindeutig, da es sich sowohl um eine klinisch manifestierte Infektion als auch um einen Durchseuchungstiter handeln kann.

Für die eindeutige Sicherung der Diagnose bleibt daher nur der Weg, den Erreger aus den verschiedenen Organen oder Körperflüssigkeiten zu isolieren oder spezifische Immunreaktionen gegen *B. burgdorferi* nachzuweisen. Ein Erregernachweis aus Patientenmaterial ist insbesondere im Frühstadium (EM), bei dem ein Antikörpernachweis häufig negativ ist, zu empfehlen. Die Anzüchtung von Borrelien bedarf jedoch eines komplexen Mediums, viel Erfahrung bei der Kultivierung und ist sehr zeitintensiv, da bis zu fünf Wochen für den Erregernachweis benötigt werden.

Mit dem Einsatz der empfindlichen Polymerase-Ketten-Reaktion (PCR) könnte der direkte Erregernachweis diagnostisch verwertbar werden. Leider konnte die PCR bisher nur in Tierversuchen erfolgreich eingesetzt werden; für einen diagnostischen Einsatz am Menschen ist sie jedoch noch nicht genügend evaluiert. So wiesen Schwartz et al. (1992. J. Clin. Microbiol. 30:3082-3088) nach, dass die PCR im Vergleich zur herkömmlichen Kultivierungstechnik nur unwesentlich bessere Ergebnisse erbrachte. Die Sensitivität, gemessen an Seren von Frühpatienten, betrug mit PCR 59-62%, während der Kulturnachweis in 57% der Fälle gelang. Die hohe Sensibilitat der PCR bringt es jedoch mit sich, dass - wie bei allen anderen Methoden- auch hier falsche Ergebnisse möglich sind (Berger, 1994. Dermatol. Clin. 12:19-24).

Nachdem die direkten Nachweismethoden zu zeitaufwendig sind, werden in der Regel serologische Tests durchgeführt, wobei sich für die Routineuntersuchung der Immunfloureszenz-Assay (IFA), der ELISA und die Immunblot-Analyse durchgesetzt haben. Da der IFA sehr subjektiv und arbeitsintensiv ist, wurde er vom ELISA abgelöst. Für die serologische Diagnose der Lyme Borreliose sind daher ELISA und Western Blot die am weitesten verbreiteten Testsysteme. Der ELISA-Test hat den Vorteil, dass er automatisierbar und daher gut zum "Screenen" grosser Probenmengen geeignet ist. In der Frage der Spezifität erwies sich der Western Blot als besser, wenngleich dieser Test arbeitsintensiver ist und bei der Auswertung viel Erfahrung voraussetzt. Vor allem in der sehr sensiblen Frühphase der Lyme Borreliose wird dem Western Blot eine höhere Detektionsrate zugesprochen (Aguero-Rosenfeld et al., 1993. J. Clin. Microbiol. 31:3090-3095; Stiernstedt et al., 1991. Scand. J. Infect. Dis. Suppl. 77:136-142; Satz, 1992. Schweiz. med. Wochenschr. 122:1779-1791; Schmitz et al., 1993. Eur. J. Clin. Microbiol. Infect. Dis. 12:419-424). Ein wichtiger Unterschied beider Testsysteme besteht darin, dass beim ELISA mehr native Epitope bestimmt werden können als beim Western Blot.

Der Nachteil eines Ganzzell-Lysat-ELISA-Tests besteht darin, dass er nur angibt, ob Antikörper mit den im ELISA verwendeten Antigenen reagieren oder nicht. Es werden dabei einerseits konformative Epitope detektiert, aber es ist andererseits nicht möglich zu unterscheiden, ob es sich bei dieser Reaktion um eine spezifische Reaktion oder um eine Kreuzreaktion handelt. Dies führt daher oft zu falsch positiven Ergebnissen. Ein Problem der Spezifität der Tests sind breite Kreuzreaktionen des Erregers *B. burgdorferi* zu anderen bakteriellen Erregern, wie orale Spirochäten, anderen *Borrelien* und insbesondere zu *T. palladium,* dem Erreger der Syphilis. Da die Antigene im allgemeinen aus Lysaten des gesamten Erregers bestehen, werden auch Antikörper gegen "common antigens" erfasst. Um bei serologischen Tests die Kreuzreaktivität zu reduzieren, werden die zu untersuchenden Seren daher häufig mit *T. phagedenis, B. hermsii* oder *E. coli* absorbiert. Mit diesen Methoden sollen die wichtigsten kreuzreagierenden Antikörper aus den Seren entfernt werden.

Die Nachteile des Western Blot bestehen darin, dass die meisten konformativen Epitope nicht detektiert werden können und bisher noch keine Standardisierung möglich ist.

Angesichts der oben angeführten Vor- und Nachteile beider Testsysteme wurde immer öfter vorgeschlagen, den ELISA als "Screening-Assay" und den Western Blot als "Confirmatory-Assay" anzuwenden (Stanek, 1991. Infection. 19:263-267; Rose et al., 1991. Pediatrics. 88:465-470; Grodzicki et al., 1988. J. Infect. Dis. 157:790-796). Obwohl diesen Vorschlägen entsprechend schon kommerziell Kombinations-Testkits auf der Basis des ELISA und des Western Blot erhältlich sind, verfügt kein Test über eine ausreichende Spezifität und Sensitivität (Bakken et al., 1992. JAMA. 268:891-895). Christenson et al. (1991. J. Clin. Lab. Anal. 5:340-343) stellten fest, dass die Sensitivität der ELISA-Testsysteme in der Frühphase der Lyme Borreliose bei 57-71% und bei Spätphasepatienten bei 86-100% liegt.

Die Ergebnisse eines serologischen Tests sind stark davon abhängig, welches Testantigen verwendet wird. Bei den meisten Tests werden als Antigene ganze *B. burgdorferi*-Zellysate oder Gesamtzell-Ultrasonifikate eingesetzt. Da die meisten publizierten Tests nur Organismen der Spezies *B. burgdorferi* sensu stricto, insbesondere Stamm B31, verwenden, die Heterogenität der einzelnen Spezies jedoch immer wieder betont wird (van Dam et al., 1993. Clin. Infect. Dis. 17:708-717; Wilske et al., 1994. Med. Microbiol. Immunol. 183:43-59), müssen Resultate hinsichtlich der Spezifität bestimmter Antigene immer vorsichtig interpretiert werden. Ein weiteres Problem bei der Verwendung von Ganzzell-Lysaten, insbesondere beim Western Blot, ist die Überladung von Antigenen in bestimmten Bereichen, wodurch nicht einwandfrei entschieden werden kann, welches Antigen reagiert hat.

Aufgrund der Probleme, die Ganzzell-Lysate mit sich bringen, wurde versucht, nur jene Borrelienantigene in serologischen Tests zu berücksichtigen, die möglichst hoch spezifisch sind und darüber hinaus regelmässig auftreten. Bisher konnte jedoch kein Antigen gefunden werden, das diesen Forderungen genügt (Satz, 1992. Schweiz. med. Wochenschr. 122:1779-1791).

Es konnten zwar einige weit verbreitete Oberflächenproteine mit immunogenem Charakter gefunden werden (Osp A, OspC); es zeigte sich aber, dass diese (i) von den einzelnen Stämmen unterschiedlich stark exprimiert werden und (ii) auch innerhalb der einzelnen Subspezies in verschiedene Serotypen aufgeteilt werden. So lässt sich das Lipoprotein OspA in mindestens 7 verschiedene Serotypen auftrennen (Wilske et al., 1993. J. Clin. Microbiol. 31:340-350). Aufgrund von RFLP-Analysen lässt sich das Lipoprotein OspC in mindestens 35 Serotypen auftrennen (Livey et al., 1995. Mol. Microbiol. 18: 257-269). Bei den verschiedenen Untersuchungen zeigte sich, dass eine Reaktion mit OspA spezifisch ist, dennoch tritt sie in der Regel selten und wenn, dann erst in der Spätphase der Lyme Borreliose auf (Engstrom et al., 1995. J. Clin. Microbiol. 33:419-427).
Vor allem in der Frühphase der Erkrankung werden Antikörper gegen OspC gebildet. Untersuchungen zeigten jedoch, dass OspC Sequenzhomologien mit Antigenen von *B. hermsii* aufweist und zwischen den Antigenen Kreuzreaktionen beobachtet werden (Carter et al., 1994. Infect. Immun. 62:2792-2799). OspC kommt trotzdem bei der serologischen Diagnostik eine grosse Bedeutung zu, da es ein sehr gutes Immunogen ist und eine Antikörperreaktion nachgewiesen werden kann.

WO 95/15781 beschreibt die Verwendung von OspC Antigen von B. burgdorferi Stamm 2591 (B. burgdorferi sensu stricto) als einziges Immunogen zur Detektion von anti-B. burgdorferi Antikörpern in Patienten mit einer eindeutig positiven EM.

Wilske et al. (1994. Med. Microbiol. Immunol. 183:43-59) stellten fest, dass bei der Austestung von rekombinantem OspC der IgG-Antikörper, jedoch nicht der IgM-Antikörper, stark speziesspezifisches Verhalten zeigt. IgM Antikörper scheinen daher konservative Epitope von rekombinantem OspC detektieren zu können. Auch Gerber et al. (1995. J. Infect. Dis. 171:724-727) schlugen daher vor, rekombinantes OspC für die Diagnose der frühen Lyme Borreliose einzusetzen. Sie fanden in verschiedenen Testsystemen (Ganzzell-Lysat-ELISA, Immunblot und IgM rOspC ELISA) mit Seren von Patienten mit einer eindeutigen EM eine Sensitivität bis zu 46% und eine fast 100%ige Spezifität. Sie merken jedoch an, dass erst der Nachweis erbracht werden muss, dass auch IgM-Antikörper von Patienten mit keinen oder anderen klinischen Manifestationen der frühen Lyme Borreliose mit rOspC reagieren, um die Sensitivität dieses Tests zu bestätigen.

Die Tatsache, dass es bei OspC und OspA mehrere Subtypen gibt, erschwert den Einsatz dieser Proteine in einem diagnostischen Kit, da darin theoretisch alle unterschiedlichen Subtypen vereint werden müssten. Da Seren von Lyme Borreliose-Patienten zumeist Antikörper gegen nur eine der drei bekannten Spezies haben, schlugen Theisen et al. (1993. J. Clin. Microbiol. 31:2570-2576) vor, in einem serodiagnostischen Test OspC Antigen von allen drei *B. burgdorferi* Gruppen (*B. burgdorferi sensu stricto, B. garinii, B.afzelii*) einzusetzen.

Neben OspA und OspC wurden die Proteine P100 (identisch mit P83 oder E90), 41 kD und P39 als immundominante Proteine beschrieben und in einem Diagostikum verwendet. Reaktionen gegen P100 sind zwar bei Frühphasen-Patienten zu finden, doch werden entsprechende Antikörper in der Regel erst im weiteren Krankheitsverlauf gebildet. Antikörper gegen das 41 kD Antigen treten zwar sehr früh auf, doch zeigte sich, dass Reaktionen gegen dieses Antigen selbst nicht spezifisch für *B. burgdorferi* sind, da Kreuzreaktionen mit anderen Organismen, wie *E. coli* oder *Treponemen*, beobachtet wurden (Luft et al., 1993. Res. Microbiol. 144:251-257; Ditton et al., 1992. FEMS Microbiol. Lett. 94:217-220). Beim P39 handelt es sich um ein Protein, das vor allem in der Frühphase der Erkrankung von Antikörpern detektiert wird (Ma et al., 1992. J. Clin. Microbiol. 30:370-376; Aguero-Rosenfeld et al., 1993. J. Clin. Microbiol. 31:3090-3095). Alle fünf genannten Proteine wurden bisher auch auf rekombinantem Weg hergestellt und gegen verschiedene Patienten-und Kontrollseren getestet (Wilske et al., 1994. Med. Microbiol. Immunol. 183:43-59; Fawcett et al., 1993. J. Rheumatol. 20:734-738).

Die Ergebnisse bisheriger Untersuchungen verschiedener Testsysteme führten jedoch zu der Schlussfolgerung, dass ein diagnostischer Kit auf serologischer Basis nicht nur auf einem Testantigen beruhen kann und dass die Sensitivität durch die Verwendung von Antigenen von verschiedenen Spezies von *B. burgdorferi sensu lato* erhöht werden kann (Wilske et al. 1994. Proceedings of the VI Internationale Conference on Lyme Borreliosis, Bologna 19-22.06., 179-182). Wilske et al. kombinierten daher rekombinant hergestellte immundominante Antigene (P100, p41, OspA, OspC, p41i) von repräsentativen Stämmen von *B. burgdorferi* sensu lato in einem Immunblot. Sie stellten fest, dass die Spezifität durch den Einsatz dieser rekombinanten Proteine zwar erheblich erhöht wird, die Sensitivität aber im Vergleich zu Ganzzell-Lysaten deutlich geringer ist und die Detektionsrate von spezifischen IgM oder IgG Antikörpern mit dem jeweiligen spezifischen Protein variierte. Bei der Anwendung von rekombinanten Antigenen zeigten sich zudem erhebliche Unterschiede zwischen den einzelnen Spezies. So reagierte P100 bei einer Studie mit Seren von Neuroborreliose-Patienten unterschiedlich, je nachdem von welcher Spezies es exprimiert wurde (Wilske et al., 1994. Med. Microbiol. Immunol. 183:43-59).

Durch die starke Variation im Reaktionspattern von Stämmen verschiedenen Ursprungs (Haut etc.) oder Ländern (Nordamerika oder Europa) stellte sich die Frage, inwieweit die Heterogenität von europäischen Stämmen die serologischen Tests beeinflusst. Insbesondere ist zu erwähnen, dass in kommerziellen Testkits vorwiegend Antigene von *B. burgdorferi* sensu stricto Stamm B31 verwendet werden.

Eine Reihe von im Western Blot auffälligen Antigenen im Molekulargewichtsbereich von 19 kD, 22 kD, 29 kd, 34 kD, 35 kD, 60 kd, 66 kD und 74 kD wurde ebenfalls identifiziert (Coleman et al., 1992. J. Infect. Dis. 165:658-666; Wallich et al., 1993. Infect. Immun. 61:4158-4166; Lam et al., 1994. Infect. Immun. 62:290-298; Luft et al., 1991. J. Immunol. 146:2776-2782; Norris et al., 1992. Infect. Immun. 60:4662-4672; Bergström et al., 1992. Scand. J. Infect. Dis. 24:181-188; Scopio et al., 1994. J. Bacteriol. 176:6449-6456). EP 0 465 204 offenbart antigene Proteine von *B. burgdorferi* B31 mit einem Molekulargewicht von 25, 28, 38, 44, 48, 52, 54, 58, 60, 68, 80 und 90 kD (+-3kd), die in einem diagnostischen Kit verwendet werden können. WO 90/04411 beschreibt immunologisch aktive Fraktionen von *B. burgdorferi* B31 und ACA, die Antigene mit verschiedenen Molekulargewichten beinhalten.

Wie oben erwähnt, treten damit sowohl beim Einsatz von rekombinanten Antigenen als auch von Ganzzell-Lysat-Antigenen Schwierigkeiten mit der Sensitivität bzw. Spezifität der Antigenreaktionen auf. Insbesondere bei der Verwendung von Ganzzell-Lysaten wurde für die Auswertung von Immunoblots versucht, durch geeignete Auswertungskriterien, die Spezifität und die Sensitivität des Testverfahrens zu erhöhen. So wurde von Dressler et al. (1993. J. Infect. Dis. 167: 392-400) zur Auswertung von Western Blots bei der Serodiagnose sogenannte diskriminierende Testkriterien entwickelt, wobei ein positives Ergebnis durch das Auftreten von mindestens 2 von 8 möglichen Banden im Molekulargewichtsbereich von 18, 21, 28, 37, 41, 45, 58 und 93 kD bei IgM und von mindestens 5 von 10 möglichen Banden im Bereich von 18, 21, 28, 30, 39, 41, 45, 58, 66 und 93 kD bei IgG im Immunblot angezeigt wird.

Ma et al. (1992. J. Clin. Microbiol. 30: 370-376) fanden im Immunblot signifikante Antigene von *B. burgdorferi* sensu stricto (Stamm B31) im Molekulargewichtsbereich von 94, 83, 75, 66, 60, 55, 46, 41, 39, 43, 31, 29, 22 und 17 kD.

Engstrom et al. (1995. J. Clin. Microbiol. 33:419-427) benutzten für serologische Untersuchungen *B. burgdorferi* sensu stricto Stamm 297 und schlugen als positive Kriterien für IgM eine Immunreaktion mit 2 von 3 Proteinen (24, 39 und 41 kD) und für ein positives IgG mit 2 von 5 Proteinen (20, 24, 35, 39 und 88 kD) vor. Es wird jedoch einhellig von der Fachwelt darauf hingewiesen, dass für die Beurteilung der Serodiagnose bei Lyme Borreliose, insbesondere bei der Auswertung von Immunblots, keine befriedigenden Kriterien zur Verfügung stehen.

Für die Problematik, dass Patienten zwar ein positives klinisches Bild, jedoch eine negative Serologie aufweisen, konnten bislang keine geeigneten Verfahrenskriterien definiert werden. Auch gibt es zur Zeit noch keine befriedigende diagnostische Interpretation für jene Menschen, die zwar kein klinisches Bild, jedoch eine positive Serologie aufweisen (inapparente oder stumme Infektion). Obwohl zum gegenwärtigen Zeitpunkt mehrere serologische Methoden bekannt sind, gibt es noch keinen Test, der eine zufriedenstellende Sensitivität und Spezifität besitzt und auch in der Lage ist, auf serologischer Basis zwischen Früh-und Spätmanifestation zu unterscheiden. Weiterhin gibt es bisher kein serologisches Testsystem, das eine Unterscheidung zwischen den verschiedenen Spezies und Serotypen von *B. burgdorferi* nach einem Infekt zulässt. Ebenso steht bisher kein standardisierter Western Blot zur Verfügung, der einen verbesserten Nachweis von Lyme Borreliose erlaubt. Dies gilt insbesondere im Hinblick auf die bestehenden Probleme mit methodischen Varianten, der Variabilität der *B. burgdorferi* Stämme und der Komplexität der Antigene.

Es besteht daher ein dringender Bedarf für einen verbesserten serologischen Test mit einer hohen Sensitivität und Spezifität zur Bestimmung einer Serokonversion bei Lyme Borreliose, sowie einer Bestimmung der Spezies des Infekts, um rechtzeitig therapeutische Massnahmen einzuleiten. Es besteht weiter ein dringender Bedarf für die Bereitstellung von *B. burgdorferi* spezifischen Antigenen zur Entwicklung eines diagnostischen Test für Lyme Borreliose, der möglichst Antigene enthält, die (1) von alle pathogenen *B. burgdorferi* Stämmen exprimiert werden, (2) hohe Antigenität mit detektierbaren Antikörpern im frühen Stadium der Immunantwort besitzen und (3) keine Kreuzreaktivität mit nicht-*B. burgdorferi* spezifischen Antigenen aufweisen. Bei der Entwicklung eines Diagnostik-Tests ist daher die Auswahl der Antigene, die eine hohe Sensitivität und Spezifität für den Nachweis im frühen Infektionsstadium aufweisen, von entscheidender Bedeutung.

Ziel der vorliegenden Erfindung ist es daher, ein verbessertes Verfahren gemäss der oben erwähnten Art zur Verfügung zu stellen, welches eine erhöhte Sensitivität und Spezifität aufweist, einfach zu standardisieren ist und zum Nachweis von Lyme Borreliose und zum Erstellen einer Serodiagnose auch in einem frühen Infektionsstadium sowie zur Bestimmung des Serotyps des verursachenden Infekts verwendet werden kann. Ein solcher Test sollte auch die Möglichkeit bieten, Prognosen für auftretende Symptome der Krankheit aufzustellen.

Die vorstehende Aufgabe wird erfindungsgemäss gelöst durch Zurverfügungstellung eines diagnostischen Mittels, welches als reagierende Komponenten mindestens 4, vorzugsweise mindestens 6, am meisten bevorzugt mindestens 8, der von einer Membranfraktion von B. afzelii abgeleiteten Antigene P870, P412, P402, P365, P315, P310, P280, P251, P230 und P180 umfasst. Es ist bevorzugt, dass das erfindungsgemässe diagnostische Mittel sämtliche der vorstehend genannten Antigene beinhaltet. Sofern das erfindungsgemässe diagnostische Mittel nicht sämtliche der genannten Antigene beinhaltet oder wenn einige der Antigene durch andere ausgetauscht sind, so mag damit immer noch ein ausreichend spezifischer Test auf Lyme Borreliose vorliegen. Ein optimales Ergebnis ist allerdings bei Vorliegen sämtlicher der genannten Antigene zu erwarten.

Überraschenderweise wurde gefunden, dass die in einer Membranfraktion von *B. afzelii* Stamm Orth enthaltenen Antigene mit einem Molekulargewicht von 87,0 kD, 41,2 kD, 40,2 kD, 36,5 kD, 31,5 kD, 31,0 kD, 28 kD, 25,1 kD, 23 kD und 18 kD diskriminierende Antigene darstellen, die spezifisch mit anti-*B. burgdorferi* Antikörpern reagieren und keine Kreuzreakivität mit anderen, nicht Lyme Borreliose spezifischen Antikörpern aufweisen. Durch die erhöhte Spezifität und Sensitivität der im erfindungsgemässen diagnostischen Mittel enthaltenen Antigene ist daher ein verbesserter Nachweis einer Serokonversion bei Verdacht auf Lyme Borreliose möglich.

Die Identifizierung der diskriminierenden Antigene erfolgte durch eine bisher noch nicht beschriebene Analyse der Molekulargewichte der Proteine einer Membranfraktion von *B. afzelii*. Dazu wurde eine Membranfraktion II von *B. afzelii* präpariert, die sich insbesondere dadurch auszeichnet, dass sie ein ausgewogenes Verhältnis von verschiedenen Antigenen enthält, wodurch eine Überladung von Proteinen mit gleichem oder ähnlichem Molekulargewicht, wie sie bei Ganzzell-Lysaten auftritt, vermieden wird. Eine Präparation von *B. burgdorferi*-Zellen besteht aus mehreren hundert verschiedenen Proteinen. In einer Präparation einer einzelnen Membranfraktion von *B. burgdorferi* wurden im Rahmen dieser Erfindung insgesamt 63 verschiedene Proteine identifiziert. Die Proteine der Membranfraktion wurden über vorher genau ermittelte, standardisierte Bedingungen in einem Gelelektrophoresesystem aufgetrennt und die Molekulargewichte bestimmt. Erst die verbesserten Analysebedingungen erlaubten eine eindeutige und reproduzierbare Zuordnung von Proteinen zu einem entsprechenden Molekulargewicht.

In einem weiteren Schritt wurden die Proteine mittels einer Serie von monoklonalen Antikörpern identifiziert, so dass eine Unterscheidung zwischen schon bekannten und noch nicht identifizierten Antigenen möglich war. Dabei wurde festgestellt, dass drei der *B. afzelii* Proteine äquivalent zu schon bekannten Antigenen anderer B. burgdorferi Stämme sind: P870 ist äquivalent zu Protein P100 bzw. E90 von Stamm PKo; P365 ist äquivalent zu Protein 35E, und P251 ist äquivalent zu OspC.
Zur Identifizierung solcher Antigene, die bei einer Immunreaktion eine Unterscheidung zwischen kreuzreaktiven und spezifischen Antigenen erlauben, wurde ein Panel von Seren von Lyme Borreliose Patienten mit verschiedenen Syndromtypen, von Syphilis-Patienten und eine Blutspende-Kontrollgruppe gegen nach Molekulargewicht aufgetrennte Proteine der Membranfraktion II von *B. afzelii* getestet. Die Auswertung der Immunblots erlaubte die Identifizierung von sogenannten diskriminierenden Antigenen, die spezifisch mit anti-*B. burgdorferi* Antikörpern reagieren und deren Reaktion mit einem in einer Probe gegebenenfalls enthaltenen Antikörper die Probe in bezug auf Lyme Borreliose als serokonvertiert ausweisen. Es hat sich gezeigt, dass die als diskriminierend identifizierten Antigene der Membranfraktion II von *B. afzelii* bei einer Immunreaktion sich durch eine besonders hohe Spezifität und Sensitivität gegen *B. burgdorferi* Antikörper auszeichnen.

Gemäss einer Ausführungsform der vorliegenden Erfindung umfasst das diagnostische Mittel eine Membranfraktion von *B. afzelii*, vorzugsweise ausgewählt aus der Gruppe von *B. afzelii* Stamm Orth und Stamm H15. Der *B. afzelii* Stamm Orth ist bei der deutschen Sammlung für Mikroorganismen (DMS) unter der Nr. 11048 und der Stamm H15 unter der Nr. 11047 gemäss dem Budapester Vertrag hinterlegt.

Nach einer weiteren Ausführungsform der vorliegenden Erfindung enthält das diagnostische Mittel als Reagenskomponenten die rekombinanten Antigene P870, P412, P402, P365, P315, P310, P280, P251, P230 und P180, wobei die Antigene abgeleitet sind von Antigenen der Membranfraktion II von *B. afzelii* mit einem Molekulargewicht von 87,0 kD, 41,2 kD, 40,2 kD, 36,5 kD, 31,5 kD, 31,0 kD, 28 kD, 25,1 kD, 23 kD und 18 kD. Der Vorteil des Einsatzes von rekombinanten Antigenen liegt darin, dass (i) nur eine spezifische Bindung von Serumantikörpern mit diesen Antigenen möglich ist; (ii) es möglich ist, Mischungen von homologen Antigenen unterschiedlicher Serotypen oder Spezies herzustellen; (iii) eine Vereinfachung und/oder Standardisierung des Tests ermöglicht wird; (iv) eine höhere Sensitivität erreicht wird; und (v) das spezifische Antigen in genügender Menge im Test vorhanden ist.

Die rekombinanten Antigene können mittels bekannter gentechnologischer Verfahren hergestellt werden. Die für die Herstellung der rekombinanten Antigene eingesetzten Verfahren umfassen dabei Schritte, wie Isolieren der Antigene des entsprechenden Molekulargewichts aus dem SDS-Gel, Herstellen von monoklonalen Antikörpern, wie etwa von Lane et al. (1985. J. Immunol. Method, 81:223) beschrieben wurde, Expressions-Screenen einer Genbank von *B. afzelii* mit den monoklonalen Antikörpern und Identifizieren und Isolieren der positiven Klone, die das rekombinante Antigen exprimieren. Aus Zellen, die mit dem Antigen-exprimierenden Klon transfiziert sind, kann anschliessend das Antigen mittels gängiger Verfahren isoliert und gereinigt werden. Die gereinigten Antigene können als Reagenskomponenten im erfindungsgemässen diagnostischen Mittel zur Durchführung eines Nachweises von Lyme Borreliose eingesetzt werden.

Die gentechnische Herstellung kann mit allen gängigen prokaryotischen und eukaryotischen Expressionssystemen, wie z.B. mit Plasmiden, die eine Expression in Wirtszellen wie *E. coli, B. subtilis* oder Hefe, insbesondere in *P. pastoris* erlauben, durchgeführt werden.

Gemäss einer weiteren Ausführungsform enthält das erfindungsgemässe diagnostische Mittel als Reagenskomponenten die von einer Membranfraktion II von *B.afzelii* abgeleiteten gereinigten Antigene P870, P412, P402, P365, P315, P310, P280, P251, P230 und P180. Die Zellen eines ausgewählten Stammes von *B. afzelii*, insbesondere von *B. afzelii* Stamm Orth, werden dazu nach bekannten Verfahren lysiert, die Membranfraktion II durch selektive Zentrifugation von den übrigen Zellbestandteilen abgetrennt und die Proteine mittels eines Detergenz, vorzugsweise einem nicht-denaturierenden Detergenz, aus der Membran extrahiert. Die solubilisierten Proteine werden anschliessend mittels chromatographischer Verfahren, wie etwa Ionenaustauschchromatographie oder HPLC, gereinigt.

Im erfindungsgemässen diagnostischen Mittel liegen die Reagenskomponenten P870, P412, P402, P365, P315, P310, P280, P251, P230 und P180 vorzugsweise isoliert bzw. räumlich voneinander getrennt und an einem festen Träger immobilisiert vor. Falls das diagnostische Mittel gemäss einer Ausführungsform eine Membranfraktion von *B. afzelii* ist, werden die Proteine mittels gelelektrophoretischer Verfahren, wie etwa SDS-PAGE, nach Molekulargewicht aufgetrennt und anschliessend an einem festen Träger, z.B. an einer Membran oder einem Filter, vorzugsweise eine Nitrocellulosemembran, immobilisiert.

Nach einer besonderen Ausführungsform werden die Proteine einer Membranfraktion oder einem Gemisch, welches gereinigte Antigene enthält,in einem 1-spurigen SDS-Gel mit einer Spurbreite von z.B. 8 cm, aufgetrennt und auf eine Membran transferiert. Die Übertragung der Proteine kann durch bekannte Verfahren, wie Diffusionstechnik oder Elektrotransfer, erfolgen.

Die derart an einen festen Träger gebundenen Reagenskomponenten des diagnostischen Mittels werden anschliessend für die Durchführung eines Western Blot, vorzugsweise eines modifizierten Western-Blot in Form eines Surfblot®, eingesetzt. Die Austestung von Antikörpern gegen aufgetrennte und transgeblottete Antigene eines gewählten *B. burgdorferi* Stammes wird gemäss der vorliegenden Erfindung als Vertikalanalyse bezeichnet. Die Membran mit den gebundenen Antigenen wird dazu entweder in Streifen geschnitten, die Streifen markiert und die einzelnen Streifen mit einer Probe inkubiert, oder die Membran wird in eine Surfblot®-Apparatur eingespannt, wodurch etwa 30 verschiedene Proben gleichzeitig mit dem diagnostischen Mittel in Kontakt gebracht werden können.

Werden im diagnostischen Mittel gereinigte Antigene oder gentechnisch hergestellte rekombinante Antigene als Reagenskomponenten eingesetzt, so werden die isoliert vorliegenden Antigene direkt an einen festen Träger, ausgewählt aus Platten, Streifen, Membranen, Filter, Papier, Film oder Perlen, immobilisiert. Dazu werden die isolierten und gegebenenfalls gereinigten Antigene in einem geeigneten Puffer suspendiert und ein gewünschtes Aliquot der Suspension an den Träger gebunden.

Gemäss einer weiteren Ausführungsform werden die nach der Reinigung isoliert vorliegenden Reagenskomponenten in einem gewünschten Verhältnis gemischt und die Mischung, im wesentlichen bestehend aus den gereinigten *B. afzelii* Membranproteinen P870, P412, P402, P365, P315, P310, P280, P251, P230 und P180, über Gelelektrophorese nach dem Molekulargewicht aufgetrennt und die Antigene an einen festen Träger, vorzugsweise eine Membran oder ein Filter, gebunden. Die trägergebundenen Reagenskomponenten werden für eine Vertikalanalyse zum Nachweis von *B. burgdorferi* Antikörpern eingesetzt.

Gemäss einer weiteren Ausführungsform werden die isoliert und gereinigt vorliegenden Antigene zur Herstellung eines Line-Blot verwendet. Dazu wird ein Aliquot einer Suspension enthaltend ein diskriminierendes Antigen, als Linie, gegebenenfalls mit Hilfe einer Surfblot-Apparatur, auf den Träger aufgebracht und immobilisiert.

Gemäss einer weiteren Ausführungsform werden die isoliert vorliegenden Antigene an die Oberfläche von Mikrotiterplatten gebunden, wobei die einzelnen Reagenskomponenten vorzugsweise in separate Mulden der Platte aufgebracht werden. Es kann jedoch auch eine Mischung aller Antigene an die Oberfläche einer Plattenmulde gebunden werden. Die Austestung der Proben kann dann gemäss bekannter ELISA-Testverfahren durchgeführt werden, wobei jeweils Aliquote einer zu testenden Probe, beispielsweise ein Humanserum, zu jeder Reagenskomponente gegeben und anschliessend eine chromogene Substratreaktion durchgeführt wird.

Gemäss der vorliegenden Erfindung erfolgt die Durchführung des Immunassays der Vertikalanalyse vorzugsweise als Western Blot oder ELISA. Es können jedoch auch andere im Stand der Technik bekannte Immunassays, etwa ein Enzym-, Lumino-, Fluoro- oder Radioimmunassay, zur Durchführung des erfindungsgemässen Verfahrens eingesetzt werden.

Werden zur Herstellung des erfindungsgemässen diagnostischen Mittels gereinigte Antigene eingesetzt, so kann die Konzentration der im Western Blot, im ELISA oder einem anderen Immunassay vorliegenden Reagenskomponenten einfach standardisiert werden, da die Antigenkonzentration vorher ermittelt, gegebenenfalls in einem beliebigen Verhältnis gemischt, und auf den festen Träger aufgebracht werden kann.

Gegebenenfalls enthält das erfindungsgemässe diagnostische Mittel als weitere Reagenskomponenten mindestens ein OspC Antigen, vorzugsweise mindestens 4, besonders bevorzugt mindestens 10, am meisten bevorzugt mindestens 15, im wesentlichen abgeleitet von den Borrelien-RFLP-Typen 1, 2, 5, 7, 8, 9, 10, 12, 13, 14, 15, 16, 17, 19, 20, 21, 22, 23, 25 und 34.

Für einen optimalen Test ist es bevorzugt, dass dem erfindungsgemässen diagnostischen Mittel als weitere Reagenzkomponenten alle OspC Antigene der vorstehend genannten Borrelien-RFLP-Typen zugegeben werden. Es ist auch möglich, dass in dem diagnostischen Mittel gemäss der Erfindung eines oder mehrere dieser Antigene fehlen oder durch andere ersetzt sind.

Das erfindungsgemässe diagnostische Mittel der vorliegenden Erfindung wird insbesondere zur Durchführung eines Immunassays zum Nachweis von anti-*B. burgdorferi*-Antikörpern verwendet.

Ein anderer Aspekt der vorliegenden Erfindung umfasst einen Testkit zum Nachweis von *B. burgdorferi* Antikörper in einer Vertikalanalyse, enthaltend ein diagnostisches Mittel der oben beschriebenen Art, Reagenzien und Lösungen zur Durchführung einer Detektion eines Antigen/Antikörperkomplexes, ein Auswertungssystem zum Erstellen einer Serodiagnose und Instruktionen sowie gegebenenfalls eine Surfblot®-Apparatur.

Liegt das diagnostische Mittel gemäss einer besonderen Ausführungsform als Surfblot-Membran vor, so lassen sich damit bis zu 30 verschiedene Proben gleichzeitig testen. Da für die Durchführung eines Surfblot eine eigene Apparatur notwendig ist, enthält der erfindungsgemässe Testkit gegebenenfalls eine Surfblot® oder eine analog funktionierende Apparatur. Reagenzien und Lösungen enthalten im erfindungsgemässen Testkit Puffer, wie z. B. TBS-Tween® 20 oder PBS-Tween-20®, Enzym-konjugierte Antikörper, Enzymsubstrat, positive und negative Referenzen, Reagenzkontrollen sowie Kontrollstreifen. Vorzugsweise enthält der Testkit eine Folien-Schablone, die entsprechende Markierungen an der Laufposition der diskriminierenden Antigene aufweist, und ein Auswertesystem.

Gemäss einer weiteren Ausführungsform enthält der erfindungsgemässe Testkit gegebenenfalls ein diagnostisches Mittel, enthaltend als Reagenskomponenten mindestens ein OspC Antigen, vorzugsweise mindestens 4, besonders bevorzugt mindestens 10, am meisten bevorzugt mindestens 15, das (die) im wesentlichen von den Borrelien-RFLP-Typen 1, 2, 5, 7, 8, 9, 10, 12, 13, 14, 15, 16, 17, 19, 20, 21, 22, 23, 25 und 34 abgeleitet sind. Diese Reagenskomponenten können ebenfalls an einen festen Träger sein.

Bisher wurde von der Fachwelt vorgeschlagen, OspC Antigene, abgeleitet von jeweils einem Vertreter der Spezies von *B. burgdorferi* sensu stricto, *B. afzelii* und *B. garnii* oder idealerweise von allen Vertretern aller bekannten Serovar- oder RFLP-Typen in einem diagnostischen Test einzusetzen. Im Rahmen der vorliegenden Erfindung wurde OspC Antigen, abgeleitet von allen bisher bekannten 35 RFLP-Typen gegen ein repräsentatives Panel von Humanseren getestet, und es wurde dabei überraschenderweise gefunden, dass Patientenseren mehrheitlich mit mindestens einem von 20 ausgewählten RFLP-Typen reagieren. Durch den Einsatz von OspC Antigen, abgeleitet von 20 ausgewählten Borrelien-RFLP-Typen als Reagenskomponenten, in einem diagnostischen Mittel ist es daher erstmals möglich, mit einer serologischen Methode eine frühe Immunantwort bei Infektion mit *B. burgdorferi* unabhängig vom Serotyp des Erregers nachzuweisen. Obwohl bekanntermassen in einer frühen Phase der Infektion Antikörper gegen OspC gebildet werden, war es bisher aufgrund der Heterogenität von OspC nicht immer möglich anti-OspC-Antikörper im Immuntest nachzuweisen. Erst durch die erfindungsgemässe Auswahl einiger OspC-RFLP-Typen und ihre Verwendung als Reagenskomponenten in einem diagnostischen Mittel ist es möglich, ein System mit hoher Sensitivität und Spezifität zur Verfügung zu stellen, wobei jedoch ein Minimum an OspC Antigen-Varianten eingesetzt werden muss. Dies ist insbesondere im Hinblick auf die wirtschaftliche Herstellung eines hochsensitiven diagnostischen Tests entscheidend, da theoretisch alle RFLP-Typen enthalten sein müssten.

Ein weiterer Aspekt der vorliegenden Erfindung betrifft daher auch ein diagnostisches Mittel, enthaltend als Reagenskomponenten mindestens 4 OspC Antigene, vorzugsweise mindestens 10, besonders bevorzugt mindestens 15, im wesentlichen von den Borrelien-RFLP-Typen 1, 2, 5, 7, 8, 9, 10, 12, 13, 14, 15, 16, 17, 19, 20, 21, 22, 23, 25 und 34, das für einen Immunassay zum Nachweis von anti-*B. burgdorferi* Antikörpern und/oder zum Bestimmen des Serotyps des Borrelien-Erregers eingesetzt werden kann. Für einen optimalen Test ist es bevorzugt, dass das erfindungsgemässe diagnostische Mittel alle OspC Antigene der vorstehend genannten Borrelien-RFLP-Typen umfasst.

Das erfindungsgemässe diagnostische Mittel ist gemäss einer Ausführungsform der Erfindung eine Membranfraktion, welche im wesentlichen von den Borrelien-RFLP-Typen 1, 2, 5, 7, 8, 9, 10, 12, 13, 14, 15, 16, 17, 19, 20, 21, 22, 23, 25 und 34 abgeleitet ist. Ausgewählte Vertreter dieser RFLP-Typen sind in Tabelle 1 aufgelistet. Es können natürlich auch andere Vertreter der erfindungsgemäss ausgewählten RFLP-Typen, die von Livey et al. (1995. Mol. Microbiol. 18: 257-269) beschrieben werden, als Quelle der Reagenskomponenten eingesetzt werden.

Werden für die Herstellung des diagnostischen Mittels Membranfraktionen der ausgewählten Borrelien-RFLP-Typen eingesetzt, so wird von einzelnen Repräsentanten-Stämmen die Membranfraktion isoliert, die Membranproteine gelelektrophoretisch getrennt, die Proteine auf einen festen Träger immobilisiert und die immobilisierten Reagenskomponenten zur Durchführung eines Nachweises von anti-*B. burgdorferi* Antikörpern oder zur Bestimmung des Serotypes eingesetzt. OspC Antigen kann aber auch durch Isolierung und Reinigung der Proteine aus Borrelien, wie in EP 0 522 560 beschrieben, gewonnen werden.

Gemäss einer bevorzugten Ausführungsform wird OspC Antigen, abgeleitet von ausgewählten RFLP-Typen von Borrelien, gentechnisch hergestellt und als rekombinantes Antigen im diagnostischen Mittel eingesetzt. Livey et al. (1995. Mol. Micobiol. 18:257-269) offenbart die Aminosäure- und Nukleotidsequenzen von OspC-RFLP-Typen, die für die Herstellung der verschiedener OspC Antigenvarianten mittels bekannter gentechnischer Verfahren verwendet werden können. Gereinigtes oder rekombinant hergestelltes Osp-C Antigen wird in einem geeigneten Puffer gelöst und an einem Träger immobilisert. Wird als fester Träger eine Membran, vorzugsweise eine Nitrocellulosemembran eingesetzt, so erfolgt die Aufbringung auf die Membran in Form von einzelne Tropfen der Lösung (Dot-Blot) oder als Linien (Line-Blot). Das so hergestellte diagnostische Mittel kann dann entsprechend zur Durchführung einer Horizontal-Western-Blot-Analyse eingesetzt werden. Eine weitere Ausführung umfasst die Aufbringung der OspC Antigene auf eine feste Trägersubstanz, vorzugsweise in Form von Mikrotiterplatten, wobei die einzelnen Reagenskomponenten jeweils in einer Plattenmulde immobilisiert werden. Gegebenenfalls kann auch eine Mischung von einigen oder allen gereinigten Antigenen an die Oberfläche einer Vertiefung gebunden werden.

Die vorliegende Erfindung umfasst desweiteren die Verwendung eines diagnostischen Mittels enthaltend als Reagenskomponenten OspC Antigene, im wesentlichen abgeleitet von den Borrelien-RFLP-Typen 1, 2, 5, 7, 8, 9,10, 12, 13, 14, 15, 16, 17, 19, 20, 21, 22, 23, 25 und 34, zum Nachweis von *B. burgdorferi* Antikörper und/oder zum Bestimmen des Serotyps des Borrelien-Erregers.

Das erfindungsgemässe diagnostische Mittel wird gemäss einem weiteren Aspekt der Erfindung in einem Testkit zur Verfügung gestellt, der zusätzlich Reagenzien und Lösungen zur Durchführung einer Detektionsreaktion der Bildung eines Antigen/Antikörperkomplexes sowie ein Auswertungssystem zum Erstellen einer Serodiagnose und Instruktionen dazu enthält.

Als Reagenskomponenten im diagnostischen Mittel werden gemäss der vorliegenden Erfindung von einer Membranfraktion von *B.afzelii* abgeleitete Antigene, im wesentlichen P870, P412, P402, P365, P315, P310, P280, P251, P230 und P180, und/oder OspC Antigene, im wesentlichen abgeleitet von den Borrelien-Typen 1, 2, 5, 7, 8, 9, 10, 12, 13, 14, 15, 16, 17, 19, 20, 21, 22, 23, 25 und 34, verwendet, wobei die erfindungsgemässen Mittel jeweils unabhängig voneinander in separaten Immun-Testssystemen zum Nachweis von *B. burgdorferi*-Antikörpern eingesetzt werden können.

Erfindungsgemäss umfasst der diagnostische Test mindestens 4 der vorstehend genannten Antigene, bevorzugt sämtliche oder im wesentlichen sämtliche der genannten Antigene.

Zur Stellung einer Serodiagnose werden vorzugsweise beide Testssysteme eingesetzt, da ein Positivnachweis unter Umständen nur bei einem Testsystem möglich ist.

Gemäss einer Ausführungsform der Erfindung wird das diagnostische Mittel, enthaltend diskriminierende Antigene, abgeleitet von der Membranfraktion II von *B. afzelii* in einer Vertikalanalyse, vorzugsweise einer Western Surfblotanalyse, und das diagnostische Mittel, enthaltend OspC-RFLP-Antigenvarianten in einer Horizontalanalyse, vorzugsweise einer Line-Blot-Analyse, verwendet. Durch die Kombination beider Analysesysteme, Vertikal-und Horizontalanalyse, wird eine höhere Sensitivität und Spezifität erzielt, sodass auch solche Proben als positiv oder negativ eingestuft werden können, bei denen bisher kein eindeutiges Ergebnis möglich war. Dadurch bietet das vorliegende Verfahren den Vorteil, dass insbesondere falsch negative, aber auch durch Kreuzreaktionen auftretende falsch positive Resultate ausgeschlossen werden können. Es können jedoch sowohl für die Durchführung der Vertikalanalyse als auch der Horizontalanalyse andere im Stand der Technik bekannte Immunassays, etwa ein Enzym-, Lumino-, Fluoro- oder Radioimmunassay, eingesetzt werden.

Gemäss einer weiteren Ausführungsform werden die gereinigten Reagenskomponenten, und zwar sowohl der diskriminierenden Antigene als auch der ausgewählten OspC-RFLP-Typen, in einem ELISA-Test verwendet, indem die isolierten und gereinigten Antigene jeweils getrennt, gegebenenfalls in einer gewünschten Mischung, an die Oberfläche einzelner Vertiefungen von Mikrotiterplatten gebunden und Aliquote einer zu testenden Probe mit jeder Reagenskomponente in Kontakt gebracht werden. Dadurch ist eine einfache Kombination von beiden Immun-Testsystemen in Form eines ELISA-Tests möglich.

Die vorliegende Erfindung umfasst daher auch einen Kombinationstestkit aus einem diagnostischens Mittel, welches im wesentlichen die von einer Membranfraktion II von B. afzelii abgeleiteten Antigene P870, P412, P402, P365, P315, P310, P280, P251, P230 und P180 enthält, zur Durchführung einer Vertikalanalyse und einem diagnostischen Mittel, welches als Reagenskomponenten mindestens ein OspC Antigen, vorzugsweise mindestens 4, besonders bevorzugt mindestens 10, am meisten bevorzugt mindestens 15 OspC Antigene, abgeleitet von den Borrelien-RFLP-Typen 1, 2, 5, 7, 8, 9, 10, 12, 13, 14, 15, 16, 17, 19, 20, 21, 22, 23, 25 und 34 enthält, zur Durchführung einer Horizontalanalyse zum Nachweis von *B. burgdorferi* Antikörper.

Ein anderer Aspekt der vorliegenden Erfindung betrifft einen Immunassay zum Nachweis von anti-*B. burgdorferi* Antikörpern, wobei ein diagnostisches Mittel, enthaltend als Reagenskomponenten im wesentlichen von einer Membranfraktion von *B. afzelii* abgeleitete Antigene P870, P412, P402, P365, P315, P310, P280, P251, P230 und P180, zur Verfügung gestellt und mit einer Probe, die in Verdacht steht, *B. burgdorferi*-Antikorpern zu enthalten, unter Bedingungen inkubiert wird, die die Bildung eines Antikörper/Antigen-Komplexes erlauben und Bestimmen der Anwesenheit von anti-*B. burgdorferi* Antikörpern durch Detektion eines Reaktionsproduktes, bestehend aus einem Antigen/Antikörper-Komplex, insbesondere der Komplexbildung eines Antikörpers mit einer der Reagenskomponenten.

Ein anderen Aspekt der vorliegenden Erfindung betrifft einen Immunassay zum Nachweis von anti-*B. burgdorferi* Antikörpern und/oder Bestimmung des Serotyps des Borrelien-Erregers, wobei ein diagnostisches Mittel, enthaltend als Reagenskomponenten mindestens 4 OspC Antigene, abgeleitet von den Borrelien-RFLP-Typen 1, 2, 5, 7, 8, 9, 10, 12, 13, 14, 15, 16, 17, 19, 20, 21, 22, 23, 25 und 34, bereitgestellt und mit einer Probe inkubiert wird, die möglicherweise anti-*B. burgdorferi* Antikörper enthält und Bestimmen der Anwesenheit von anti-*B. burgdorferi* Antikörpern in der Probe durch Detektion der Bildung eines Antigen/Antikörper-Komplexes, insbesondere der Komplexbildung eines Antikörpers mit einer OspC-RFLP-Typ Antigenkomponente.

Proben, die mit dem Verfahren getestet werden sind humanen oder tierischen Ursprungs, insbesondere Blut, Plasma, Serum, Liquor oder Synovial-Flüssigkeit.

Bisher wurde für die serolgische Diagnose von Lyme Borreliose ein Zwei-Testsystem vorgeschlagen (National Committee for Clinical Laboratory Standard, Second National Conference on Serologic Diagnosis of Lyme Disease held October 27-29, 1994), wobei der vorläufige Nachweis einer Infektion mit einem EIA- oder IFA-Test erfolgen sollte und die als positiv identifizierten Individuen durch einen Western Blot bestätigt werden. Für den mit dem EIA oder IFA-Test als negativ ermittelten Individuen wurde es nicht für notwendig befunden, einen weiteren Test durchzuführen.

Im Rahmen der vorliegenden Erfindung wurden 670 Humanseren getestet und festgestellt, dass eine anti-*B. burgdorferi* spezifische Immunantwort in beiden Testsystemen (Vertikalanalyse oder Horizontalanalyse), in manchen Fällen jedoch auch nur in einem der Immuntests auftreten kann. Es wurde gefunden, dass negative Ergebnisse einer Probe in einem Test, sogenannte Non-Responder, nicht zwangsläufig seronegativ sind, da sie bei der Testung im zweiten Test sehr wohl eine Immunantwort zeigen können. Damit werden die negativen Ergebnisse eines Tests entweder mit dem zweiten Test bestätigt oder nachgewiesen, dass das erste Testsystem ein falsch-negatives Resultat lieferte und die Individuen gemäss dem zweiten Immuntest zur Responder-Gruppe gehören. Der erfindungsgemässe Test wird daher sowohl in der Vertikalanalyse als auch der Horizontalanalyse als "Konfirmations-Assay (Bestätigungstest)" durchgeführt, da falsch negative Resultate ausgeschlossen und positive Resultate in einem weiteren Test bestätigt werden. Wie oben schon erwähnt, wurden durch bisherige Diagnoseverfahren die als negativ eingestuften Seren nicht in einem Konfirmation-Assay weiter getestet. Es hat sich jedoch überraschenderweise herausgestellt, dass eine Vielzahl von Proben, die über bisher bekannte Verfahren als negativ bewertet wurden, durch das erfindungsgemässe Verfahren, als positiv identifiziert werden konnten. Dies ist insbesondere in der frühe Phase der Infektion von Bedeutung, da entsprechende therapeutische Massnahmen gesetzt werden können, damit eine Manifestation der Krankheit verhindert wird. Ebenso wurden durch das erfindungsgemässe Verfahren auch solche Proben als positiv identifiziert, die bisher trotz Spätmanifestation der Krankheit als seronegativ definiert wurden. Die Diagnose von Patienten mit sogenannter "seronegativer Lyme Borreliose" gestaltete sich bisher sehr schwierig und war mit herkömmlichen serologischen Bestimmungsmethoden nicht möglich. Das erfindungsgemässe Verfahren ermöglicht daher eine empfindliche Bestimmung der Serokonversion bei Patienten in einem frühen Infektionsstadium sowie in Fällen, in denen bisher keine eindeutige Zuordnung in seropositiv oder seronegativ möglich war. Zudem ist es mit dem erfindungsgemässen Verfahren möglich, Borrelien-spezifische Antikörper in Proben unterschiedlichsten Ursprungs, insbesondere aus verschiedenen geographischen Regionen, mit sehr hoher Sensitivität und Spezifität zu detektieren, da die im Immunassay eingesetzten Antigenkomponenten mit nahezu allen bekannten Serotypen von Borrelien reagieren.

Im Rahmen der vorliegenden Erfindung wurden ebenfalls neue Serokonversionsparameter definiert, die insbesondere in Grenzfällen, bei denen bisher keine eindeutige Zuordnung in positiv oder negativ möglich war, eine bessere Bewertung ermöglichen. Das Messen der Parameter der Serokonversion erfolgt gemäss der vorliegenden Erfindung in einem standardisierten Immun-Test-System.

Serokonversionsparameter zur Bestimmung einer positiven Serokonversion wurden gemäss der vorliegenden Erfindung wie folgt definiert:

### I. Vertikalanalyse mit anschliessender Horizontalanalyse:

a) Weist eine Probe, beispielsweise ein Serum, eine IgM oder IgG-Reaktion mit mindestens einer Antigenreagenskomponente in der Vertikalanalyse auf, wobei die Reagenskomponente ein von einer Membranfraktion von *B. afzelii* abgeleitetes P870, P412, P402, P365, P315, P310, P280, P251, P230 und P180 Antigen ist, so gilt sie in bezug auf Lyme-Borreliose als serokonvertiert.
b) Weist eine Probe keine Reaktion nach a) auf, so wird sie in einer Horizontalanaylse gegen ein diagnostisches Mittel, enthaltend als Reagenskomponente OspC Antigen, im wesentlichen abgeleitet von den Borrelien-RFLP-Typen 1, 2, 5, 7, 8, 9, 10, 12, 13, 14, 15, 16, 17, 19, 20, 21, 22, 23, 25 und 34, getestet.

### II. Horizontanalyse mit anschliessender Vertikalanalyse:

a) Weist eine Probe eine IgM oder IgG-Reaktion mit mindestens einer Antigenreagenskomponenten in der Horizontalanalyse auf, wobei die Reagenskomponente ein OspC Antigen, im wesentlichen abgeleitet von den Borrelien-RFLP-Typen 1, 2, 5, 7, 8, 9, 10, 12, 13, 14, 15, 16, 17, 19, 20, 21, 22, 23, 25 und 34 ist, so gilt sie in bezug auf Lyme Borreliose als serokonvertiert.
b) Weist eine Probe keine Reaktion nach a) auf, so wird sie in einer Vertikalanalyse gegen ein diagnostisches Mittel, enthaltend als Reagenskomponente ein von einer Membranfraktion von *B. afzelii* abgeleitetes P870, P412, P402, P365, P315, P310, P280, P251, P230 und P180 Antigen, getestet.

Wird in der nach I. b) oder II.b) getesteten Probe ein Antikörper/Antigen-Komplex mit einer Antigenkomponenten nachgewiesen, so gilt die Probe als in bezug auf Lyme Borreliose als serokonvertiert.

Weist die Probe keine Reaktion nach I.b) oder II.b) auf, so wird sie gegebenenfalls nach einem bestimmten Zeitraum erneut auf eine IgM-oder IgG- Reaktion gemäss a) oder b)getestet.

Erst wenn in der Probe nach a) oder b), und gegebenenfalls nach Wiederholung des Tests, keine IgM- oder IgG-Reaktion nachzuweisen ist, gilt die Probe als eindeutig nichtserokonvertiert in bezug auf Lyme Borreliose.

Die vorliegende Erfindung umfasst daher auch ein Verfahren zum Stellen einer Serodiagnose von Lyme Borreliose, das die folgenden Schritte umfasst:
a. Bereitstellen eines diagnostischen Mittels zur Durchführung einer Vertikal- oder Horizontalanalyse,
b. Inkubieren einer biologischen Probe, die möglicherweise anti-*B. burgdorferi* Antikörper enthält, mit dem diagnostischen Mittel, und
c. Bestimmen der Anwesenheit von anti-*B. burgdorferi* Antikörpern in der Probe durch Detektion der Bildung eines Antigen/Antikörper-Komplexes, wobei die Bildung eines Komplexes zwischen einem Antikörper und einer Antigenreagenskomponente des diagnostischen Mittels für eine Serokonversion in bezug auf Lyme Borreliose indikativ ist.

Getestete Proben, die Antikörper enthalten, die in der Vertikalanalyse mit einem der Antigene P870, P412, P402, P365, P315, P310, P280, P251, P230 und P180 von *B. afzelii* oder in der Horizontalanalyse mit einem ausgewählten OspC-RFLP-Typ Antigen einen Komplex bilden, werden gemäss diesem Verfahren als Responder, also als serokonvertiert eingestuft. Die Proben, die mit einem Analyseverfahren, z.B. einer Vertikalanalyse getestet werden, werden gegebenenfalls zur Bestätigung des Resultates einem zweiten Immunassay, z.B. der Horizontalanalyse unterzogen, wodurch durch den zweiten Test gleichzeitig eine Aussage über den Serotyp des Erregers ermöglicht wird.

Die Seren, bei denen keine Antikörper-Antigen-Komplexbildung mit den diskriminierenden Antigenen der Vertikalanalyse bzw. in der Horizontalanalyse bestimmt werden kann, gelten in bezug auf den jeweilig durchgeführten Immunassay als Non-Responder und werden gegebenenfalls zur Verifizierung des Ergebnisses mit einem weiteren Immunassay getestet. Da möglicherweise in der frühen Phase der Infektion noch keine Antikörper gegen ein diskriminierendes Antigen der Membranfaktion II von *B. afzelii* gebildet worden sind, ist es besonders wichtig, Non-Responder in der Vertikalanalyse gegen ein Antigen zu testen, das insbesondere Antikörper, die in der Frühphase der Immunantwort gebildet werden, erkennt. Die Probe eines Non-Responder, getestet mit diskriminierenden Antigenen, wird daher erst nach Testen mit ausgewählten OspC Antigenen in der Horizontalanalyse als in bezug auf Lyme Borreliose als seronegativ oder seropositiv bewertet.

Falls für Patientengruppen aufgrund der Anamnese ein begründeter Verdacht einer Borrelieninfektion besteht, jedoch mit dem oben beschriebenen Verfahren in keinem der Immunassays eine Serokonversion nachzuweisen ist, sollte zur Bestätigung der Diagnose nach einem vorgegebenen Zeitraum erneut eine Immun-Testung durchgeführt werden, da bekanntermassen erst etwa 4 Wochen nach der Borrelien-Infektion spezifische Antikörper nachzuweisen sind.

Das Verfahren wird erfindungsgemäss vorzugsweise zur Serodiagnose von Lyme Borreliose und/oder zur Prognose des Krankheitssyndroms nach Borrelien-Infektion durch Bestimmung des syndrom-assozierten Antigens verwendet.

Das erfindungsgemässe Immunassay-Verfahren hat insbesondere die Vorteile, dass es (i) einfach herzustellen und standardisierbar ist, (ii) durch die Kombination von zwei Immun-Testsystemen klare Resultate liefert, wobei sowohl falsch-negative als auch falsch-positive ausgeschlossen werden, (iii) eine Vielzahl von Proben gleichzeitig und mit reproduzierbarem Ergebnis getestet werden können und (iv) für den Benutzer einfach zu handhaben und auszuwerten ist.

Im Rahmen der vorliegenden Erfindung wurden gegen die als diskriminierend identifizierten Antigene monoklonale Antikörper hergestellt. Die vorliegende Erfindung umfasst daher auch monoklonale Antikörper gegen die immunologisch aktiven Antigene P870, P412, P402, P365, P315, P310, P280, P251, P230 und P180 einer Membranfraktion von *B. afzelii* Stamm Orth. Diese monoklonalen Antikörper können in einem Immunassay eingesetzt werden, bei dem die Antikörper als Reagenskomponenten zum Nachweis von Borrelien-Antigenen in einer Probe verwendet werden.

Eine weitere Verwendung der monoklonalen Antikörper besteht darin, sie zur Reinigung der immunologisch aktiven Antigene von Borrelien einzusetzen. Dazu werden die Antikörper mit bekannten Methoden an einen festen Träger, beispielsweise eine Säulenmatrix gekoppelt, und die gebundenen Antikörper für die Affinitätschromatographie eingesetzt. Mittels dieser Affintiätschromatographie können spezifische Antigene aus Ganzzell-Lysaten von Borrelien oder aus rekombinanten Zellkulturen, die das Antigen exprimieren, gereinigt werden.

Im Rahmen der vorliegenden Erfindung wurden neue Antigene der Membranfraktion II von *B. afzelii* Stamm Orth identifiziert und über ihr Molekulargewicht charakterisiert. Die Erfindung umfasst daher auch immunologisch aktive Proteine von *B. afzelii* Stamm Orth P870, P412, P402, P365, P315, P310, P280, P230 und P180 mit einem Molekulargewicht von 87.0 kD, 41,2 kD, 40,2 kD, 36,5 kD, 31,5 kD, 31,0 kD, 28,0 kD, 23,0 kD und 18,0 kD.

### Kurze Beschreibung der Figuren:

- **Fig. 1**: Gelelektrophoretisch nach Molekulargewicht aufgetrennte Membranfraktion II von *B. afzelli* Stamm Orth. Alle Antigene, die in die Analyse einbezogen wurden, sind mit ihrem entsprechenden Molekulargewicht (in kDa) gekennzeichnet. Jene Antigene, die darüber hinaus mit bekannten monoklonalen Antikörpern identifizierbar waren, werden in der Abbildung mit Kurzbezeichnungen versehen (Osp = "Outer Surface Protein", E = "Equivalent to" gibt mit der nachfolgenden Zahl das ungefähre Molekulargewicht des entsprechenden Antigens beim homologen Stamm in kD an).
- **Fig. 2**: Anzahl der nach SDS-Gelelektrophorese bestimmbaren potentiellen Antigene der Membranfraktion II von Borrelien-Stämmen.
- **Fig. 3**: Surfblot einer Membranfraktion II von *B. afzelli* Stamm Orth, wobei an den mit kolloidalem Gold gefärbten Streifen jene Antigene markiert sind (mit Molekulargewichten oder Kurzbezeichnungen, s.o.), die sich bei der Vertikalanalyse (VA) als signifikant erwiesen.
- **Fig. 4**: Ergebnis einer Vertikalanalyse einer Auswahl von 37 Humanseren, getestet gegen die Membranfraktion II von *B. afzelii* Stamm Orth.
- **Fig. 5**: Zusammenfassung der als differenzierend und nichtredundant identifizierten Antigene der Membranfraktion II von *B. afzelii* Stamm Orth.
- **Fig. 6**: Horizontalanalyse der anti-OspC-Immunantwort von Lyme Borreliose-Patienten.
- **Fig. 7**: Spezifität der anti-OspC-Immunantwort von Humanseren.
- **Fig. 8**: Zusammenfassung des Reaktionsmuster der IgG- und IgM-Antwort von 37 Humanseren von Lyme Borreliose-Patienten nach Horizontalanalyse gegen OspC Antigenvarianten.
- **Fig. 9**: Horizontalanalyse von Humanseren gegen rekombinante OspC Antigene im Line-Blot
- **Fig. 10**: Zusammenfassung der Ergebnisse der Horizontal-und Vertikalanalyse von 190 Patientenseren von Åaland, Finnland.
- **Fig. 11**: Zusammenfassung der Ergebnisse der Horizontal-und Vertikalanalyse von 37 repräsentativen Patientenseren, Åaland, Finnland
- **Fig: 12**: Vergleich der Vertikal- und Horizontalanalyseresultate von repräsentativen Patientenseren.
- **Fig: 13**: Vergleich der Resultate der Seropositivitätskriterien von Humanseren aus 3 verschiedenen Studiengebieten
- **Fig: 14**: Detektionsraten von Seren, die entweder nur mit Vertikalanalyse oder nur mit Horizontalanalyse detektierbar sind.
- **Fig: 15**: Detektionsrate der Horizontalanalyse bei Non-responder-Seren, die bei Ausstestung in der Vertikalanalyse weder eine IgG- noch eine IgM-Reaktion aufwiesen.
- **Fig: 16**: Vergleich der Resultate von Phase I-Patienten innerhalb der Studie von Seren aus Finnland nach Analyse mittels Serokonversionskriterien.

Anhand der nachfolgenden Beispiele wird die Erfindung näher erläutert, wobei die Erfindung jedoch nicht auf die Beispiele beschränkt ist.

### Beispiel 1

### Identifizierung von diskriminierenden Antigenreaktionen von B. burgdorferi sensu lato

### a. Auswahl des Stammes und der Membranfraktion zur Bestimmung von diskriminierenden Antigenen gegen anti-B.burgdorferi Antikörper

Bei einem serologischen Testsystem werden meistens heterologe Reaktionen bestimmt, da der eine Infektion verursachende Stamm in den meisten Fällen unbekannt ist. Daher ist es folgerichtig, einen Teststamm zu wählen, der (i) möglichst viele potentielle Antigene aufweist und (ii), eine möglichst hohe Reaktivität in der Reaktion mit Antikörpern von Humanseren zeigt.

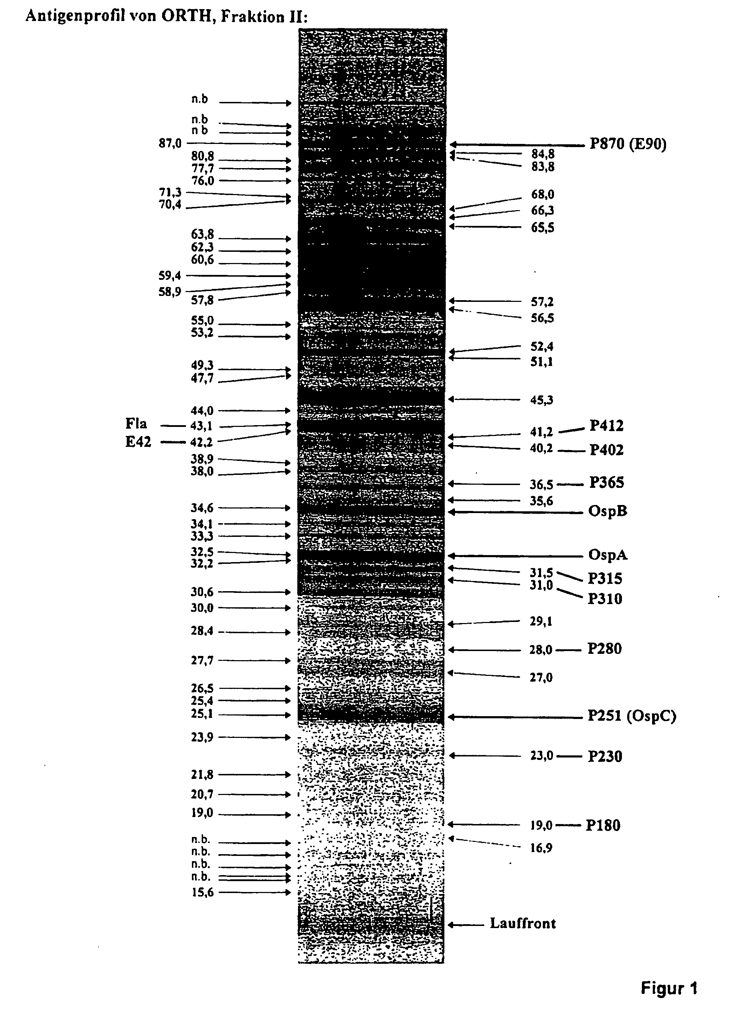

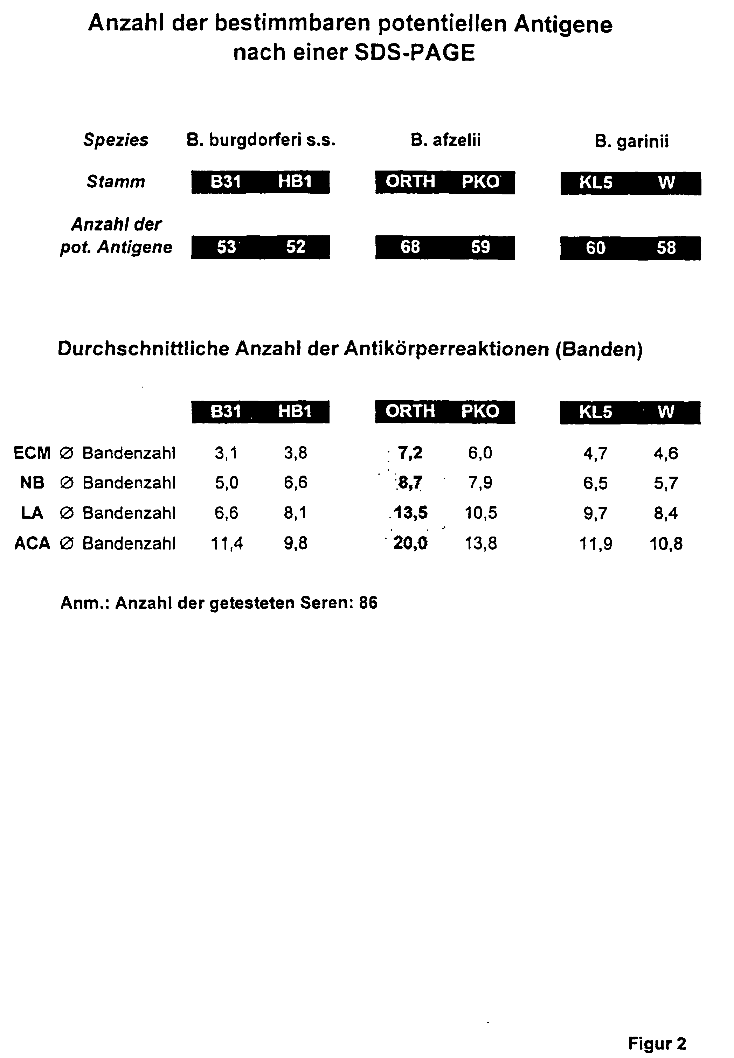

Zur Bestimmung von potentiellen Antigenen wurde die Membranfraktion II von je zwei Vertretern der Spezies *B. burgdorferi* s.s (B31, HB1), *B. afzelii*, (Orth, Pko) und *B. garinii* (K15, W) präpariert. Dazu wurden angezüchtete Zellen pelletiert, die Zellen mit einer Vibrogen®-Zellmühle aufgeschlossen, das Lysat filtriert und erneut zentrifugiert. Das Pellet, enthaltend die Membranfraktion II, wurde mit Tris/HCl gewaschen, resuspendiert und die Proteinmenge bestimmt. Die Membranfraktion II der drei Spezies wurde über eine modifizierte SDS-PAGE nach Lämmli et al. (1970. Nature 227:680-685) (Sammelgel: 3 % Acrylamid/2,6 % Bis-Acrylamid, Trenngel: 11% Acrylamid/1,8% Bis-Acrylamid, Lauflänge des Gels: 125mm) aufgetrennt. Die aufgetrennten Antigene wurden danach auf eine immobilisierende Membran übertragen und mit kolloidalem Gold gefärbt. Ein typisches Proteinmuster der Membranfraktion II des Stammes Orth zeigt Fig. 1. Die Ergebnisse der danach durchgeführten Zählung der (mit kolloidalem Gold angefärbten) potentiellen Antigene sind in Fig. 2 angeführt. Dabei zeigt sich, dass die Vertreter der Spezies *B. afzelii* und *B. garinii* eine höhere Anzahl potentieller Antigene aufweisen als die Vertreter von *B. burgdorferi* sensu stricto.

Zur Bestimmung der Sensitivität wurden 86 Seren von Lyme Borreliose Patienten mit ECM, NB, LA, ACA-Syndrom gegen oben genannte Vertreter über Surfblot (=modifizierter Western Blot) ausgetestet und die mittlere Bandenzahl (eine Bande entspricht der Bildung eines Antikörper/Antigenkomplexes) für jeden Syndromtyp ermittelt. Dabei zeigte sich, dass die Vertreter von *B. afzelii* und *B. garinii* im Durchschnitt höhere Bandenzahlen als die Verteter von *B. burgdorferi* s.s. aufwiesen (Fig. 2). Bei einer Beurteilung der Vertreter von *B. afzelii und B. garinii* in bezug auf Anzahl potentieller Antigene, und durchschnittlich auftretende Antikörper/Antigenkomplexe (Bandenzahl), erwies sich der Stamm ORTH (*B. afzelii*) in beiden Kriterien gegenüber den anderen getesten Stämmen als überlegen.

### b. Testen von repräsentativen Patientenseren mittels Vertikalanalyse (VA)

Zur Bestimmung der für die Western Blot-Vertikalanalyse signifikanten Antigene wurden 670 Humanseren aus drei europäischen Regionen in bezug auf ihre IgG- und IgM-Reaktivität gegen den Stamm ORTH (*B. afzelii*) getestet:

| Serengruppe | geographische Region | | |
|---|---|---|---|
| | CZR/Österreich | Finnland | Deutschland |
| Kontrollgruppe | 96 | 100 | 102 |
| Patientengruppe | 130 | 190 | 52 |

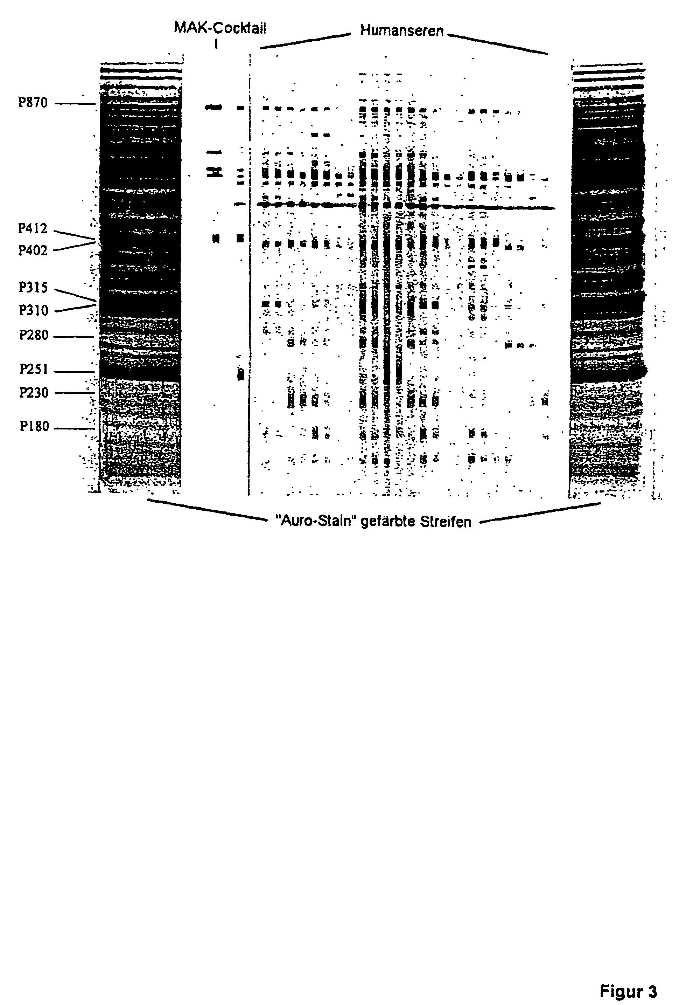

Die Durchführung der Vertikalanalyse erfolgte nach einer modifizierten Methode von Towbin et al. (1979. Proc. Natl. Acad. Sci. USA 76: 4350-4354) mittels Western Surfblot. Dazu wurden die Proteine einer Membranfraktion des Stammes Orth gemäss Beispiel 1 über ein 1-spuriges SDS-Gel aufgetrennt und die Antigene auf eine immobilisierende Membran geblottet. Danach wurden rechts und links von der Membran je 1,5 breite Streifen abgetrennt und die Streifen mit Auro-Dye® gefärbt. Der mittlere Teil der Membran wurde mit TBS-Tween 20-BSA blockiert und in eine Surfblot-Apparatur eingespannt. Die zu testenden Humanseren wurden in je eine Spurrille der Apparatur gefüllt, inkubiert, mit Puffer gewaschen und mit sekundärem Antikörper inkubiert. Für die Entwicklung wurde 5-Brom-4-chlor-3-indolylphosphat (BCIP) und Nitro-Blue-Tetrazolimchlorid (NBT) als Substrat zugesetzt und die Banden visualisiert. Fig. 3 zeigt ein typisches Western Blot-Surfblot-Muster nach Austestung mit Seren.

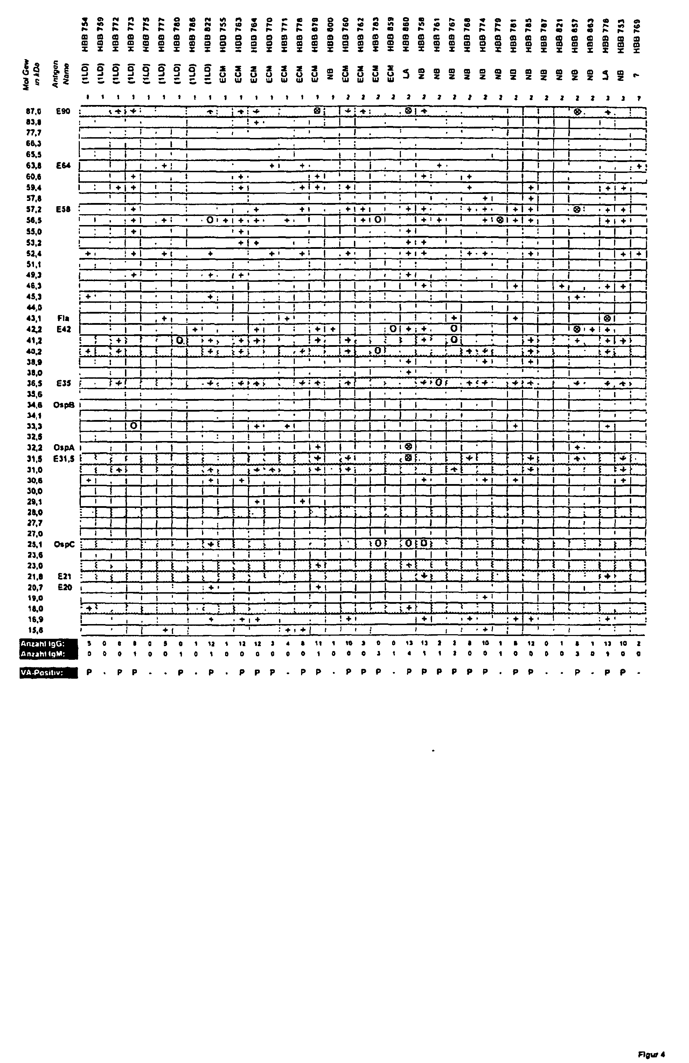

Fig. 4 zeigt die Ergebnisse der Vertikalanalyse einer Auswahl von 37 Humanseren, getestet gegen die Membranfraktion II des Stammes Orth. Links sind jene 49 Antigene (in absteigender Grösse in kD) aufgelistet, die in den verschiedenen Studien reaktiv mit Humanseren-Antikörpern waren. Bei der Auflistung wurden jene Antigene ausgespart, die nachweislich kreuzreaktive oder negativ diskriminante Reaktionen (=KRND Reaktionen) ergaben. Die Spalte neben den Molekulargewichten gibt die Namen jener Antigene an, die mit schon bekannten monoklonalen Antikörpern detektierbar waren ("E" bedeutet "Equivalent to" und gibt mit nachfolgender Zahl das ungefähre Molekulargewicht jenes Antigens an, dass durch den monoklonalen Antikörper beim homologen Stamm detektiert wurde). In der ersten oberen Zeile sind die getesteten Humanseren angeführt; in der Zeile darunter der jeweilige Reaktionstabelle sind IgG-Reaktionen mit "+", IgM-Reaktionen mit "○" dargestellt. Wurde ein Antigen sowohl durch eine IgMals auch durch eine IgG-Reaktion der Antikörper desselben Serums erkannt, so wurden diese Reaktionen durch ein "⊗" Symbol markiert. Jene Antigene, deren Reaktion mit Humanseren-Antikörpern als diskriminant bezeichnet werden, sind in der Tabelle grau unterlegt. Am Ende der Tabelle wird in zwei Zeilen die Gesamtzahl der IgG- und der IgM-Reaktionen jedes Serums angezeigt. Auch hier sind die KRND-Reaktionen nicht berücksichtigt. Die letzte Zeile gibt für jedes Serum an, wie es gemäss der Vertikalanalyse klassifiziert wurde ("P" = positiv, "-" = negativ).

### c. Statistische Analyse zur Ermittlung von diskriminierenden Antigenen

Das Ziel dieser Auswertungen war es, Antigenreaktionen zu bestimmen, die eine eindeutige Unterscheidung zwischen nicht serokonvertierten und serokonvertierten Seren ermöglichen. Antigene, die eine solche analytische Relevanz aufweisen, müssen einerseits diskriminierend sein (d.h. zwischen beiden Serokonversionszuständen unterscheiden können), andererseits sollten sie nicht redundant sein (d.h. die Reaktion sollte mit keiner anderen Antigenreaktion assoziiert sein).

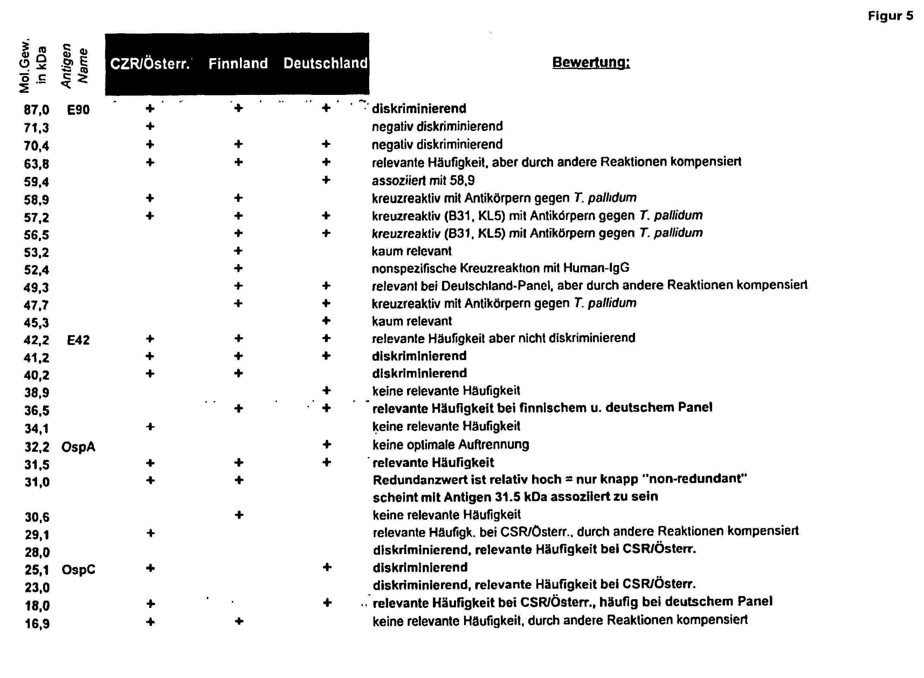

Um zunächst differenzierende von redundanten Antigenreaktionen zu unterscheiden, wurden die Surfblot-Ergebnisse jedes der drei Serenpanels aus CZ/AT, Finnland und Deutschland (insgesamt 670 Seren) über eine Redundanzanalyse ausgewertet. Das Ergebnis dieser Analyse (Figur 5a) zeigt, je nach Serenpanel, insgesamt 27 Antigene, die als differenzierend und nicht-redundant ausgewiesen wurden.

In den nachfolgend durchgeführten Analysen wurde versucht, unter besonderer Berücksichtigung der Ergebnisse der Redundanzanalyse, die diskriminierenden Antigene zu erfassen.

In den nachfolgend durchgeführten Analysen wurde versucht, unter besonderer Berücksichtigung der Ergebnisse der Redundanzanalyse, die diskriminierenden Antigene zu erfassen. Kreuzreagierende Antigene wurden erfasst, indem Seren von Syphilispatienten gegen die Stämme Orth (*B. afzelii*), B31 (*B. burgdorferi* s.s.) und KL5 (*B.garinii*), jeweils Membranfraktion II, ausgetestet wurden. In dieser Teilstudie wurden Vertreter aller drei Spezies ausgewählt, da ein möglichst breites Spektrum von kreuzreagierenden Antigenen erfasst werden sollte. Diese Untersuchung ergab für die Antigene 58.9 kDa (E60), 57.2 kDa (E58), 56.5 kDa und 47.7 kDa eine deutliche Reaktivität mit Antikörpern von Syphilis-Patienten. Daher mussten diese Antigene, die in der Redundanzanalyse noch als "differenzierend" ausgewiesen wurden, für die weiteren Betrachtungen ausgeschlossen werden.

In einem parallel dazu durchgeführten Ansatz wurden diskriminante Antigenreaktionen auf Basis der Diskriminanzanalyse ermittelt: Nachdem das tschechisch/österreichische Serenpanel hinsichtlich seiner Zusammensetzung am homogensten war (die 130 Humanseren konnten den drei klinischen Phasen der Lyme Borreliose etwa zu gleichen Anteilen zugeordnet werden), wurde dieses über die "Stepwise Logistic Regression" analysiert. Von den insgesamt 7 Antigenen, die sich aus dieser Untersuchung ergaben, musste das Antigen 58.9 kDa (E60) wegen seiner Kreuzreaktivität (mit Syphilisseren s.o.) von den weiteren Untersuchungen ausgenommen werden. Vier (87.0 kDa, 41.2 kDa, 40.2 kDa, 25.1 kD), der verbleibenden sechs Antigene stimmten mit den anderen 27 Antigenen aus der Redundanzanalyse überein. Die zwei anderen Antigene (28.0 kDa, 23.0 kDa) wurden nur über die Diskriminanzanalyse als diskriminierend bewertet, zeigten aber bei einer Abschätzung der Reaktionshäufigkeit (95%-Konfidenzintervallabschätzung) eine relevante Häufigkeit (32.8-45.3% bzw. 26.2-38.2%). Gemäss den Analysekriterien wies ein Antigen nur dann eine "relevante Häufigkeit" auf, wenn die untere Konfidenzgrenze über einem vorher festgelegten Wert von 20% lag. Zwei weitere Antigene, 71.3 kDa und 70.4 kDa (beide sind in Fig. 5 der 27 nichtredundanten Antigene enthalten) wurden über die Diskriminanzanalyse als "negativ diskriminierend" ausgewiesen. Die Antigene 58.9 kDa (E60), 47.7 kDa, 71.3 kDa und 70.4 kDa sind kreuzreaktiv oder negativ diskriminierend und wurden deshalb ausgeschlossen.

Insgesamt hatte sich durch die Anwendung der Diskriminanzanalyse die Anzahl der in Frage kommenden Antigene von 27 auf 29 (durch die Antigene 28.0 kDa und 23.0 kDa) erhöht. Durch die Kreuzreaktionsstudie mit Syphilisseren und den negativ diskriminierenden Antigenen aus der Diskriminanzanalyse reduzierte sich diese Zahl um 6.auf nunmehr 23 Antigene. Von diesen 23 Antigenen wurden bereits 6 durch die Diskriminanzanalyse bestätigt, womit noch 17 Antigene auf ihre analytische Relevanz untersucht werden mussten. Da das Antigen 52.4 kDa bei allen Seren eine nichtspezifische Reaktion mit IgG-Antikörpern zeigte, reduzierte sich die Anzahl der zu untersuchenden Antigene weiter auf 16. Diese sechzehn Antigene wurden auf die Häufigkeit ihrer Reaktionen mit Antikörpern von Humanseren untersucht. Die Abschätzung der Häufigkeit wurde durch die Berechnung der 95% Konfidenzintervalle erreicht. Auch hier wies eine Antigenreaktion eine "relevante Häufigkeit" auf, wenn der Wert der unteren Konfidenzgrenze über einem vorher festgelegten Wert von 20% lag.

In den beiden linken Spalten von Fig. 5 sind die 29 Antigene, die als differenzierend und nicht-redundant identifiziert wurden, mit ihren Molekulargewichten und Kurzbezeichnungen angeführt. Die drei anschliessenden Spalten enthalten Information zu jedem Antigen bei jedem der drei Serenpanels. Mit "+" markierte Tabellenfelder kennzeichnen jene Antigenreaktionen, die (über die Redundanzanalyse) als differenzierend bzw. non-redundant ausgewiesen wurden. Mit Ausnahme der beiden Antigene 28.0 kDa und 23.0 kDa, ist zu jedem Antigen mindestens eine "+" Markierung eingetragen. Die beiden oben angeführten Antigene wurden jedoch nicht über die Redundanzanalyse ausgewiesen, sondern durch die Diskriminanzanalyse ermittelt. Die rechte Spalte beinhaltet kurze Kommentare zu den einzelnen Antigenreaktionen. Die grau unterlegten Felder kennzeichnen jene Reaktionen, die nach allen Untersuchungen die für die Vertikalanalyse höchste analytische Relevanz aufwiesen.

Jene 10 Antigene, deren analytische Relevanz für eine Vertikalanalyse am geeignetesten schienen, sind in Fig. 5 grau unterlegt aufgelistet.

### Beispiel 2:

### Horizontale Analyse mit OspC-Varianten als Reagensantigen

### a. Selektion von Borrelien-Stämmen für die Horizontalanalyse:

Seren von 56 seropositiven Lyme Borreliose-Patienten wurden gegen Vertreter von 35 OspC-RFLP-Typen getestet. Dazu wurde von 35 RFLP-Typ Stämmen die Membranfraktion II gemäss Beispiel 1 präpariert, die Proteine mittels SDS-PAGE aufgetrennt, auf immobilisierende Membranen transferiert und eine Horizontalanalyse mit Humanseren durchgeführt. 82% der Seren reagierten mit einem oder mehreren der 35 OspC-Varianten. Basierend auf dieser Untersuchung wurden die 20 am häufigsten reagierenden Varianten ausgewählt und sind die Tabelle 1 zusammengefasst.

**Tabelle 1.**

| OspC-RFLP-Typen, die am häufigsten mit Lyme Borreliose-Seren reagieren | | |
|---|---|---|
| **Genospezies** | **Stamm** | **OspC-RFLP- Typ** |
| B.burgdorferi s.s. | ZS7 | 1 |
| B.burgdorferi s.s | IP2 | 2 |
| B. afzelii | H5 | 5 |
| B. afzelii | Orth | 7 |
| B. afzelii | H15 | 8 |
| B. afzelii | JSB | 9 |
| B. afzelii | E61 | 10 |
| B. afzelii | E51 | 22 |
| B. garinii | M57 | 12 |
| B. garinii | W | 13 |
| B. garinii | KL10 | 14 |
| B. garinii | NBSlab | 15 |
| B. garinii | IP90 | 16 |
| B. garinii | VSBM | 17 |
| B. garinii | KL11 | 19 |
| B. garinii | 20047 | 20 |
| B. garinii | NSB23a | 21 |
| B. garinii | VSDA | 23 |
| B. garinii | H8 | 25 |
| B. garinii | 153 | 34 |

Alle aufgelisteten OspC Antigen-RFLP-Typen der Tabelle 1 wurden nachfolgend in der Horizontalanalyse eingesetzt.

### b. Detaillierte Analyse der anti-OspC-Immunantwort von Patienten aus verschiedenen geographischen Regionen:

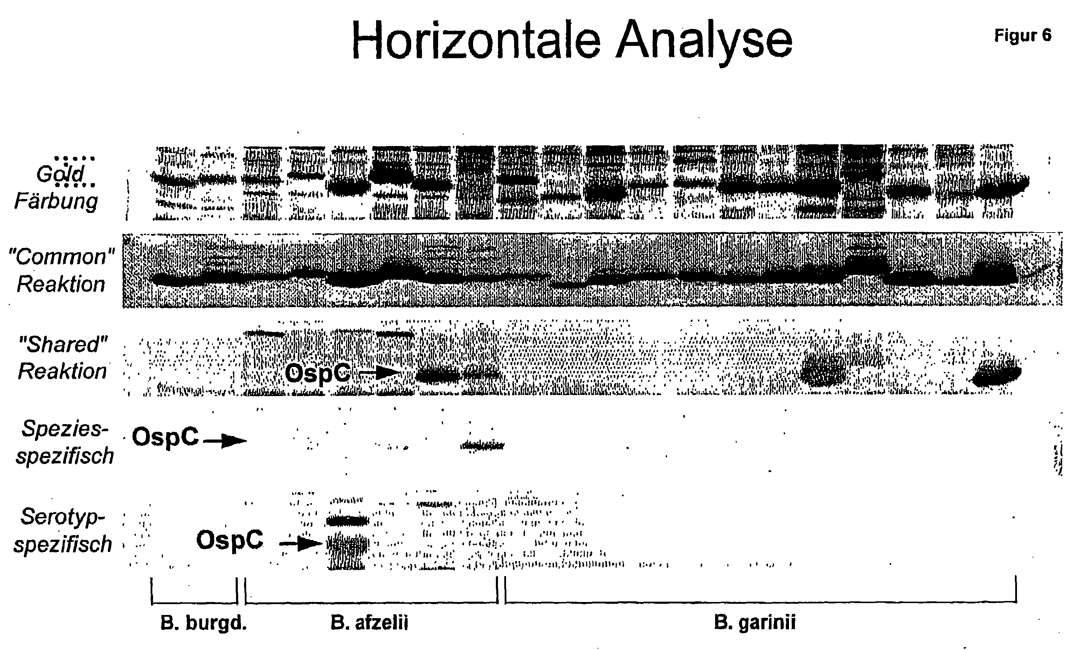

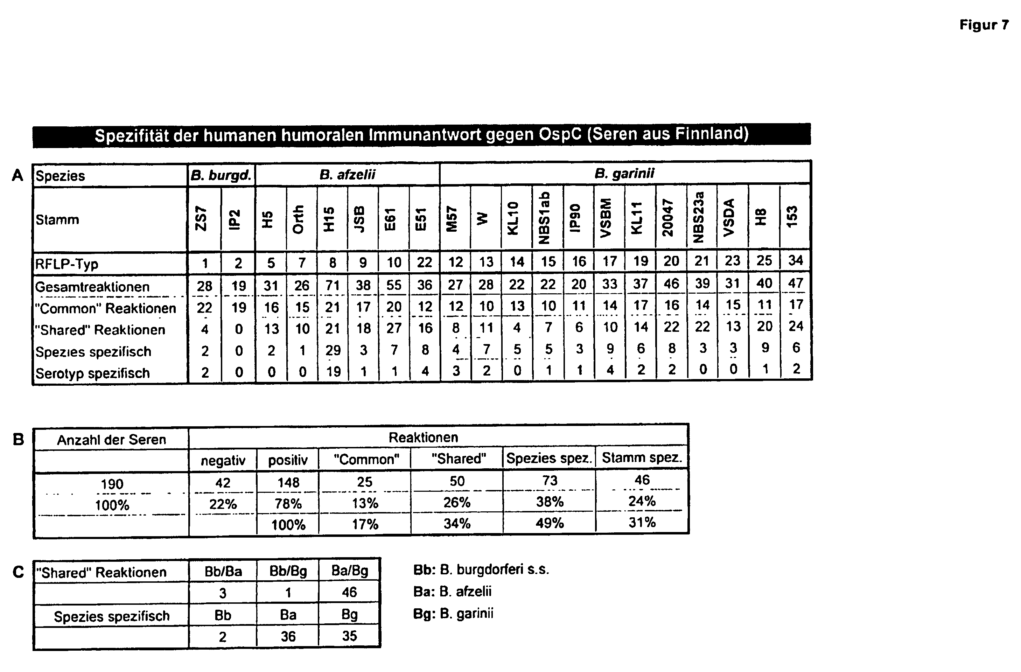

Seren von 190 Patienten von den Åaland Inseln, Finnland, die an Lyme Borreliose leiden, wurden mittels Western Blot-Horizontalanalyse (Figur 6) gegen die 20 am häufigsten reagierenden Varianten von OspC-Protein (Tabelle 1) getestet. Die Ergebnisse sind in Fig. 7 zusammengefasst. Die Spalte, die mit "Gesamtreaktionen" bezeichnet ist, gibt die Zahl der mit jedem Stamm reagierenden Seren an. Der RFLP-Typ des jeweiligen Stammes ist ebenfalls angegeben. Das OspC-Reaktionsmuster jedes einzelnen Serums ist in 3 Typen von Reaktionen eingeteilt:
Spezies-Gesamtreaktionen ("common"-Reaktionen): Seren, die mit OspC-Varianten von Stämmen aller 3 Spezies reagieren. Spezies-Teilreaktionen ("shared"-Reaktionen): Seren, die mit OspC-Varianten von Stämmen von 2 der 3 Spezies reagieren. Spezies-spezifische Reaktion (Spezies-spezifisch): Seren, die mit OspC-Varianten nur einer Spezies reagieren und daher als Spezies-spezifisch bezeichnet werden.
Stammspezifische Reaktion (Serotyp-spezifisch): Die Kategorie der Spezies-spezifischen Reaktionen wurde weiter unterteilt in solche Seren, die nur mit einem einzigen Stamm reagieren und damit als stammspezifisch gelten.
Die Kategorie der spezifischen Reaktionen ist diejenige, die die meisten Informationen bereitstellt, da sie die Spezies anzeigt, zu der der infizierende Stamm gehört. Diese Information kann für die Prognose der Krankheit von entscheidender Bedeutung sein, da es eine Beziehung zwischen dem Krankheitssyndrom, an dem der Patient leidet, und der Spezies des infizierenden Stammes gibt. *B. afzelii* kann in erster Linie aus Haut isoliert werden und ist assoziert mit einem chronischen ACA-Hautsyndrom. *B. garinii*-Stämme werden von CSF-Proben isoliert und sind meist mit Neuroborreliose assoziert, während *B. burgdorferi* sensu stricto-Stämme primär mit Lyme-Arthritis, insbesondere in Nord-Amerika, assoziert ist.

Die Zahl der Seren, die in jede der Reaktionsmuster-Kategorien fällt, ist in Figur 7 B wiedergegeben. Ungefähr 78% aller Seren zeigen eine Reaktion, wobei die prominenteste Kategorie die der "Spezies-spezifischen" ist (49% der positven Seren). Es war ebenfalls möglich, die Serotyp-Spezifität in 31% der positiven Seren anzugeben. Die Spezies-Spezifität der Seren der Kategorie "Spezies-Teilreaktion"und "Spezies-spezifische Reaktion" ist ebenfalls in Figur 7 C wiedergegeben. In beiden Kategorien wurde eine Prädominanz für Reaktionen gegen OspC Protein der Spezies *B. afzelli* und *B. garinii* festgestellt. Innerhalb der "Spezies-spezifischen Reaktion" Kategorie zeigte sich eine fast gleichmässige Verteilung zwischen den Spezies. Ermittelt man die Verteilung dieser Reaktionen unter den einzelnen OspC-Typen innerhalb der entsprechenden Spezies, so werden im Fall von *B. afzelii* fast alle Reaktionen für nur 2 RFLP-Typen, nämlich RFLP-Typ 8 (Stamm H15) und RFLP-Typ 22 (Stamm E51) beobachtet. Im Gegensatz dazu sind Spezies-spezifische Reaktionen von *B. garinii* OspC Protein gleichmässig verteilt und werden bei 9 von 12 RFLP-Typen gefunden. Fig. 6 zeigt eine typische Horizontalanalyse mit OspC-Varianten, getestet gegen verschiedene Humanseren. Die Reihenfolge der Borrelienstämme entspricht der in Fig. 7.

### c. Analyse der anti-OspC humoralen Immunantwort von individuellen Patienten

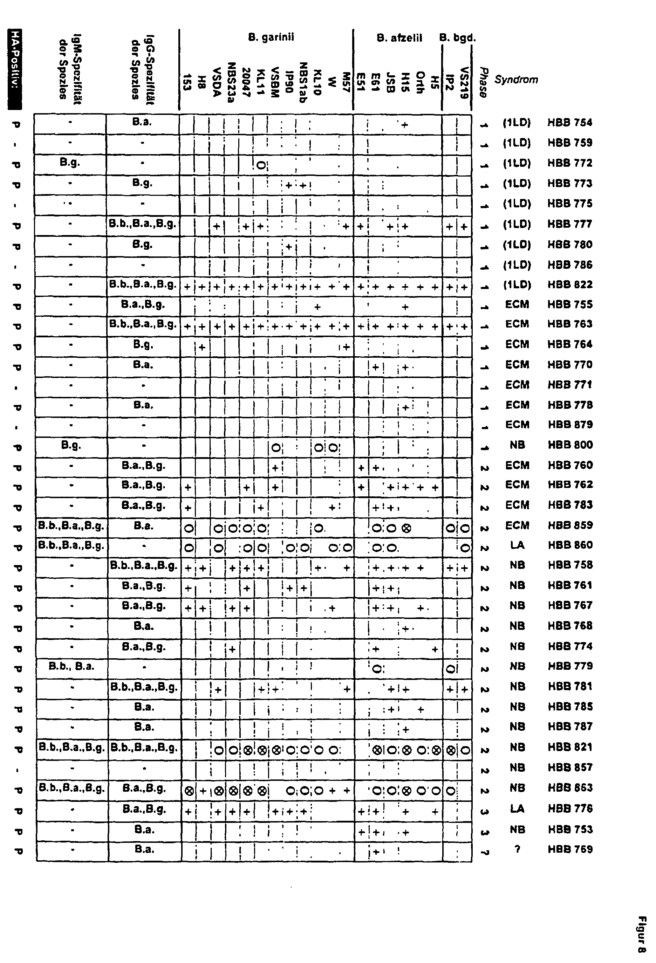

Die IgG- und IgM-Antwort von individuellen Patienten wurde in der Horizontal-Western Blot-Analyse durch Testen der Seren gegen die ausgewählten 20 OspC-RFLP-Typen bestimmt. Fig. 8 zeigt das Reaktionsmuster der IgG-und IgM-Antwort von 37 Patienten, die Teil einer grösseren Studie von Seren von 190 Patienten und 100 Blutspendern der Åaland Inseln, Finnland, war (Serenpanel gemäss Beispiel 1). Die Untersuchung wurde durchgeführt, um die Sensitivität und Spezifität der Horizontal-Western Blot-Analyse in einer stark endemischen Region zu testen. Eine positive IgG-Reaktion wurde mit "+" und eine positive IgM-Reaktion mit "○" markiert. Eine positive IgG- und IgM-Reaktion ist mit "⊗" markiert.

Zusammenfassend ist festzustellen: die Spezifität der IgG-Antwort der individuellen Patienten ist meist sehr spezifisch, da sie meist nur mit einem Stamm reagiert oder auf Stämme einer einzigen Spezies beschränkt ist. Im Gegensatz dazu ist die IgM-Antwort sehr breit und reagiert mit den meisten getesteten OspC Antigenen. Fig. 8 zeigt die Auswertung der Western Blot-Horizontalanalyse der Membranfraktion II von 20 repräsentativen Stämmen der drei Hauptspezies mit entsprechenden Patientenseren, die ebenfalls für die Vertikalanalyse gemäss Beispiel 1 eingesetzt wurden. Die unteren Spalten von Fig. 8 zeigen die "Spezies-Spezifität" der IgG- und IgM-Antwort für jedes individuelle Serum an.

### d. Testen von rekombinantem OspC von verschiedenen RFLP-Typen (Horizontalanalyse) im Surfblot

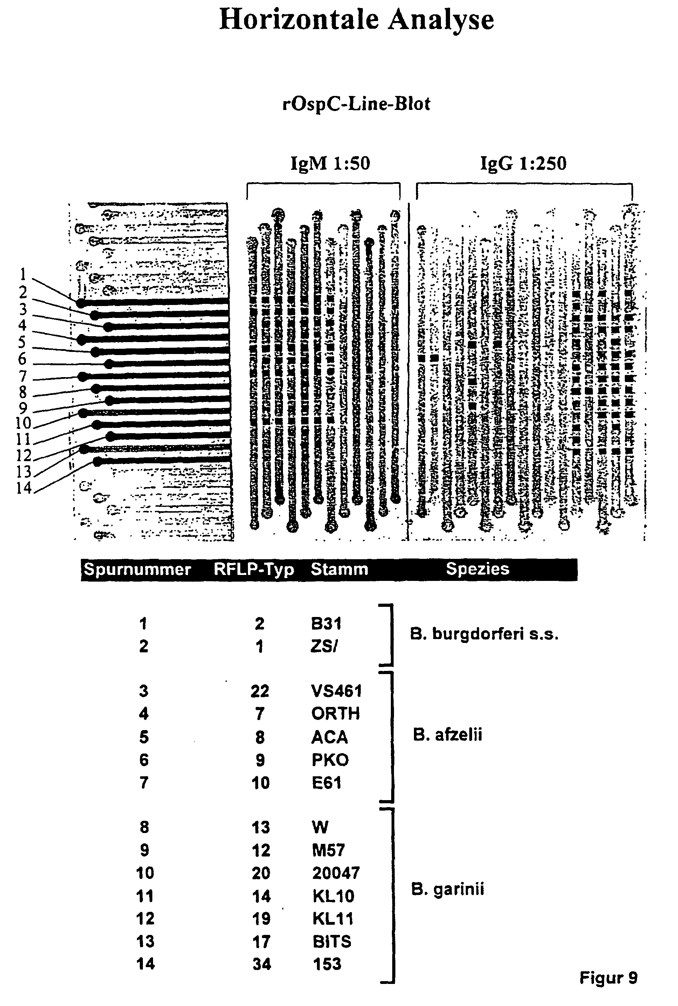

Um Antikörper gegen einzelne Membranproteine in polyspezifischen Seren nachzuweisen, wurde in dieser Nachweismethode rekombinantes OspC-Protein aus *P. pastoris* gemäss WO 94/25596 verwendet, das an Nitrocellulosemembran gebunden wurde. Im Unterschied zu herkömmlichen Western Blot-Methoden wird das rekombinante Membranprotein mit einer Surfblot-Apparatur direkt auf die Nitrocellulosemembran aufgetragen. Mit dieser Methode können pro Blot 18 OspC-Typen aufgetragen werden. Die Austestung erfolgt ebenfalls mit der Surfblot-Apparatur (30 Seren pro Blot). Danach wird der Blot mit einem Konjugat oder sekundären, konjugierten Antikörper und einer Substratlösung inkubiert. Das Reaktionsmuster eines Serums im Line-Blot (Horizontalanalyse) ist in Fig. 9 gezeigt.

### e. Testen von rekombinantem OspC von verschiedenen RFLP-Typen (Horizontalanalyse) im ELISA

Auf der Grundlage des ELISA wurden verschiedene rekombinante OspC-Proteine wie in Beispiel 2 d gegen Humanseren getestet. Die Kopplung der rekombinanten OspC Antigene an die Oberfläche der Vertiefungen einer Mikrotiterplatte erfolgte im Carbonatpuffer (pH 9,6) bei 4° C über Nacht. Nachdem unspezifische Bindungsstellen auf der Kunststoffoberfläche blockiert wurden (PBS, 10% FCS), wurden die einzelnen Seren getestet, indem Aliquote der Probe mit dem gebundenen Antigen inkubiert wurden. Zur Entfernung von ungebundenem Protein oder Antikörpern wurde mehrmals gewaschen und durch einen Test, z. B. alkalischer Phosphatase, konjugierte Antikörper nachgewiesen. Als Nullwert diente eine Kontrolle, bei der kein Antigen zugesetzt war. Als Positivkontrolle diente ein bereits ausgetestetes Positivserum bzw. als Negativkontrolle ein ausgetestetes Negativserum.

Für die Durchführung eines ELISA-Assays können gemäss der vorliegenden Erfindung folgende Varianten eingesetzt werden:
- Variante A:: Mischungen von allen OspC-RFLP-Typen werden an eine Mikrotiterplattenmulde gekoppelt.
- Variante B:: Mischungen von OspC-RFPL-Typen abgeleitet von der gleichen Spezies, z.B. *B. burgdorferi s*.*s*., *B. afzelii oder B. garinii*, werden in eine Mikrotiterplattenmulde gekoppelt.
- Variante C:: Einzelne OspC-RFLP-Typen werden in jeweils eine Mikrotiterplattenmulde gekoppelt.

Die Assay-Variante A wird für einen allgemeinen Screen auf positive Reaktivität in der Horizontalanalyse eingesetzt.
Die Assay-Variante B kann eingesetzt werden, um zwischen den generellen Kategorien der Reaktionstypen in der Humanimmunantwort zu unterscheiden. Dabei gelten die gleichen Kriterien wie in Beispiel 2 b beschrieben.
Die Assay-Variante C erlaubt die Beurteilung des Ausmasses der Kreuzreaktivität in den Testseren und in solchen Seren, die eine RFLP-Typ-Stamm-spezifische Immunantwort zeigen.

Dadurch wird eine direkte Aussage über die Spezies des OspC-RFLP-Typs des infizierenden Stammes möglich, die gegebenenfalls auch Rückschlüsse auf das Krankheitsbild des Patienten zulässt.

### Beispiel 3:

### Kombination von Vertikal- und Horizontalanalyse

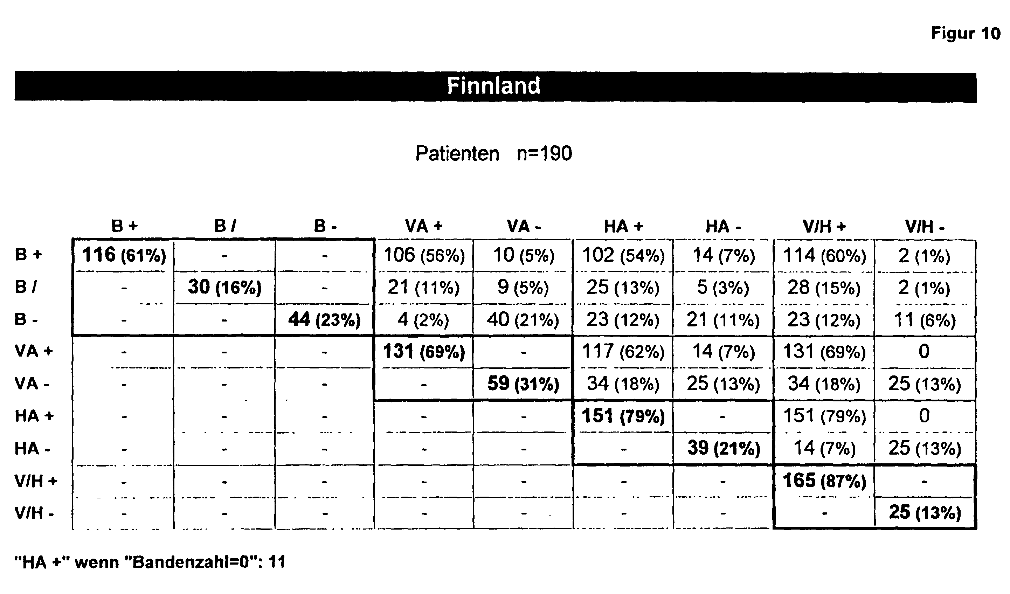

Die Testergebnisse der Vertikal-und Horizontal-Western Blot-Analyse von 190 Seren von Patienten der Åaland-Insel wurden gemeinsam ausgewertet und sind in Fig. 10 zusammengefasst. In dieser Darstellung wurden die einzelnen Analyseformen (VA und HA) hinsichtlich ihrer Effizienz miteinander verglichen. Dabei kommt sehr deutlich zum Ausdruck, dass die Kombination von Vertikal-und Horizontalanalyse die höchste Detektionsrate aufweist.

Die mit den Buchstaben "VA" überschrifteten Felder geben die Werte für die Vertikalanalyse wieder, die mit dem Buchstaben "HA", jene der Horizontalanalyse. Die Werte, die durch die Buchstabenkombination "V/H" überschriftet werden, stehen für die Ergebnisse einer Kombination aus vertikaler und horizontaler Analyse. Positive Seren sind mit "+" und negative Seren mit "-" markiert.

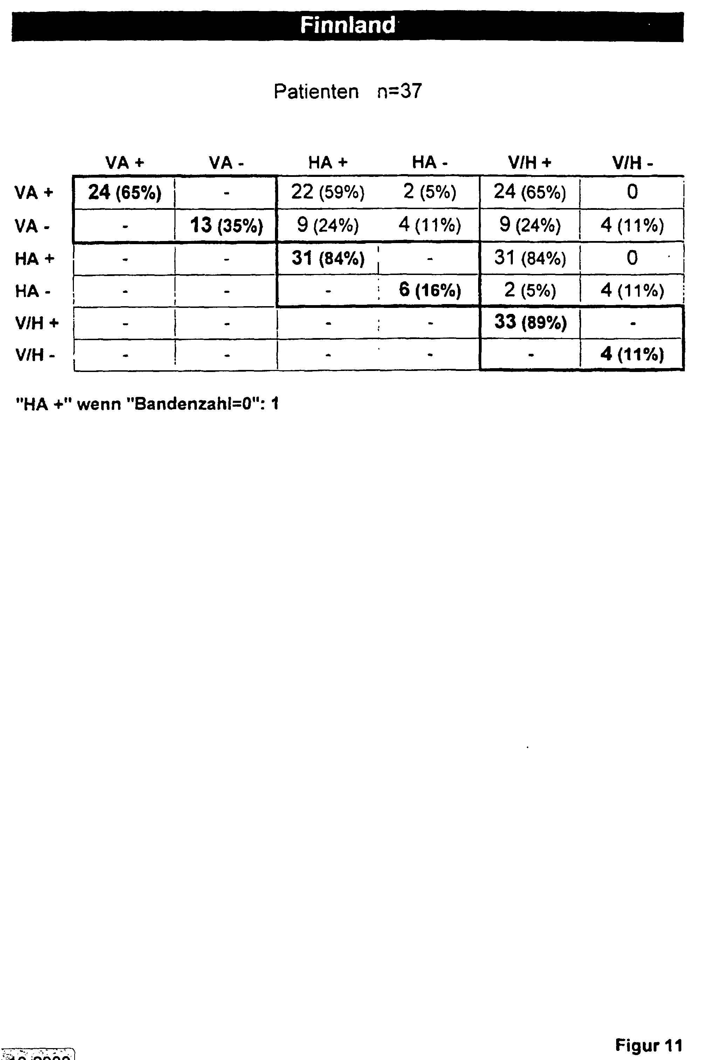

Die grau unterlegten Felder an den Kreuzungspunkten (zusammengehörende Felder sind dick eingerahmt) von identischen Zeilen- und Spaltenüberschriften geben jeweils die absolute Anzahl bzw. die Prozentzahl an Seren an. Die in Figur 10 und 11 mit dem Buchstaben "B" überschrifteten Felder geben die Werte des Bandenzahlkriteriums wieder. Dabei steht "+" für "Positiv" (≥4 Banden IgG oder ≥2 Banden IgM), "/" für "Borderline" (2-3 Banden IgG oder 1 Bande IgM), "-" für "Negativ" (0-1 Bande IgG oder 0 Banden IgM), wobei die KRND-Antigenreaktionen nicht in die Berechnung einbezogen wurden.

In Fig. 11 sind die Ergebnisse der Serenbeispiele aus den Figuren 4 und 8 dargestellt. Von den insgesamt 37 Seren konnten bei kombinierter Anwendung der Vertikal- und Horizontalanalyse 33 Seren als serokonvertiert detektiert werden. Davon konnten neun Seren nur über die Horizontalanalyse und zwei Seren ausschliesslich über die Vertikalanalyse bestimmt werden. Zwei Seren zeigten keine Reaktionen bei der Austestung gegen die Membranfraktion II des Stammes ORTH in der Vertikalanalyse. Ein Serum konnte dennoch über die horizontale Analyse als positiv bestimmt werden.

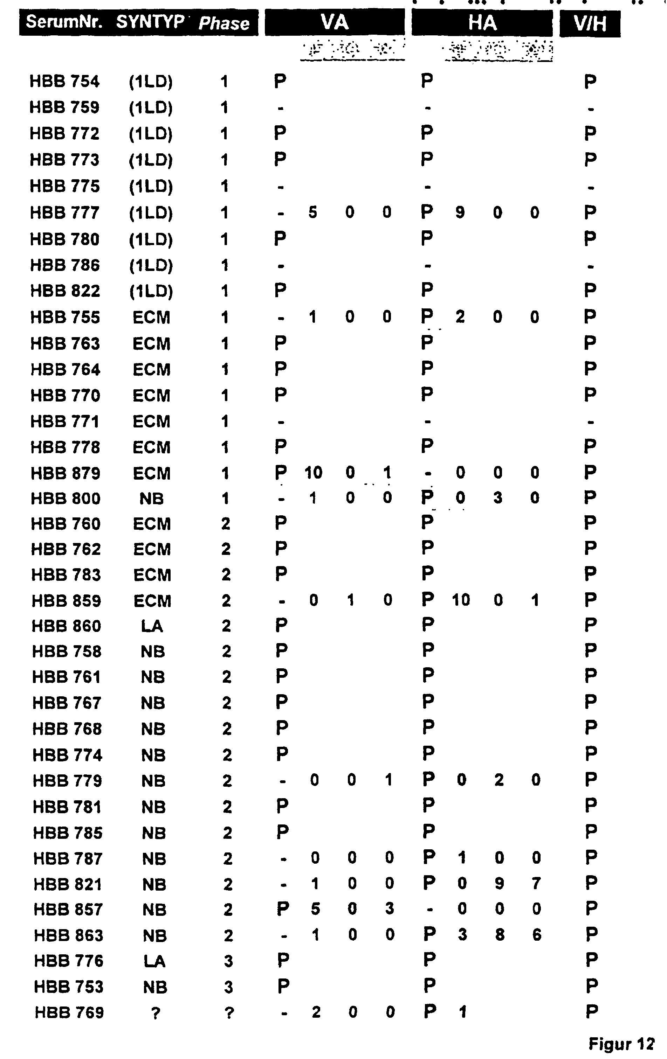

In Fig. 12 sind die Ergebnisse der beiden Beispiele für die horizontale (HA) und die vertikale Analyse (VA) vergleichend nebeneinander aufgeführt. In den linken Spalten finden sich die Humanseren, deren SYNTYPs (Syndromtyp) und die Phasenzuordnung, in derselben Reihenfolge wie in Fig. 4 und 8. Die drei nachfolgenden Spaltengruppen sind mit "VA", "HA" und "V/H" übertitelt. Die Titel sind jeweils Kurzformen der vertikalen Analyse (VA), der horizontalen Analyse (HA) oder der Kombination beider Analyseformen (V/H). Die Spaltengruppen "VA" und "HA" sind gleich aufgebaut: Die jeweils linke Spalte zeigt an, ob das betreffende Serum positiv ("P") oder negativ ("-") eingestuft wurde. Die drei anschliessenden Spalten zeigen an, wieviele Reaktionen das jeweilige Serum mit IgG-Antikörpern (+), IgM-Antikörpern (O) oder wenn sowohl eine IgG als auch eine IgM-Reaktion gegen dasselbe Antigen (Ä) gerichtet waren, aufwies. Die Spalte "V/H" gibt die Ergebnisse der Kombination beider Analyseformen wieder.

In der Tabelle sind jene Seren grau unterlegt, die entweder nur von der VA oder nur von der HA als positiv ausgewiesen wurden. Parallel dazu sind die Bandenzahlen der jeweils anderen Analyseform ausgewiesen.

Aus dieser Tabelle geht hervor, dass von den 37 getesteten Seren nur 22 sowohl von der VA als auch von der HA positiv bewertet wurden. 11 Seren wurden entweder nur von der HA (9) oder nur von der VA (2) erkannt. Bei der kombinierten Anwendung beider Bewertungskriterien wurden demnach 33 (89%) Seren detektiert. Bei den beiden Seren, die nur durch die VA bestimmbar waren zeigten sich sowohl IgM- als auch IgG-Reaktionen. Von den neun nur durch die Horizontalanalyse bestimmbaren Seren zeigten 4 Seren ausschliesslich eine IgG-, 2 Seren nur eine IgM- und 3 Seren sowohl eine IgG als auch eine IgM Reaktivität.

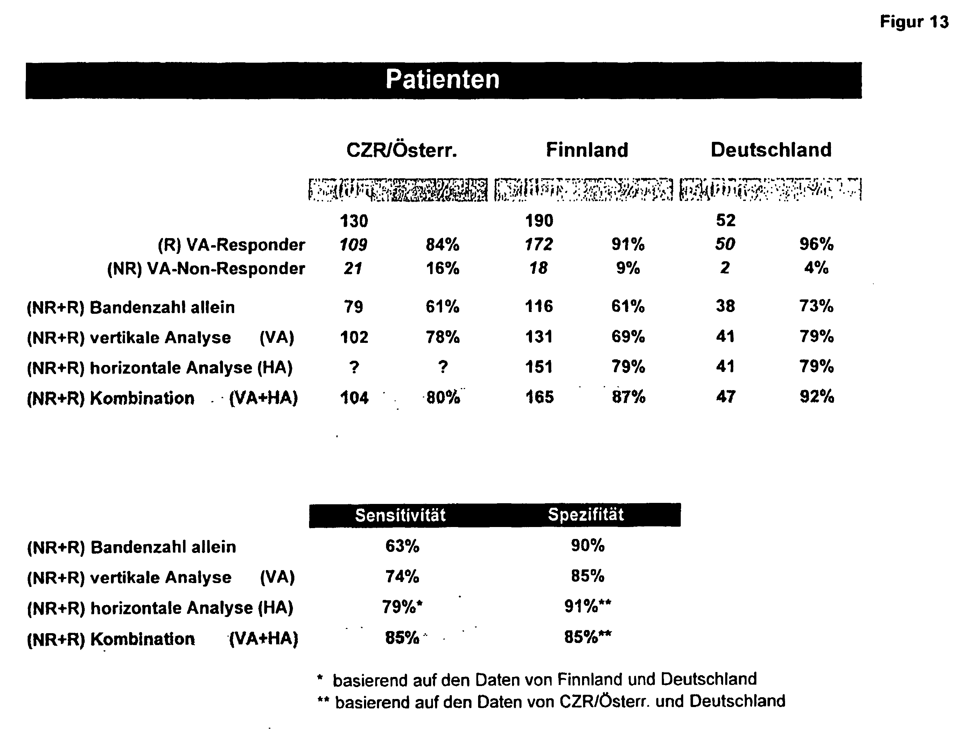

In Fig. 13 sind die Ergebnisse der drei Studien (CZR/Österreich, Finnland, Deutschland) vergleichend dargestellt. Alle Seren (Kontroll- und Patientengruppe), insgesamt 670, der drei Studien wurden über die Vertikalanalyse bewertet. Die Seren der Kontrollgruppe, aber auch die Patientenseren aus Finnland und Deutschland, wurden darüber hinaus auch über die HA klassifiziert. Bei den Patientenseren der CZR/Österreich-Studie wurden nur jene Seren über die HA bewertet, die in der VA als "negativ" befundet wurden.

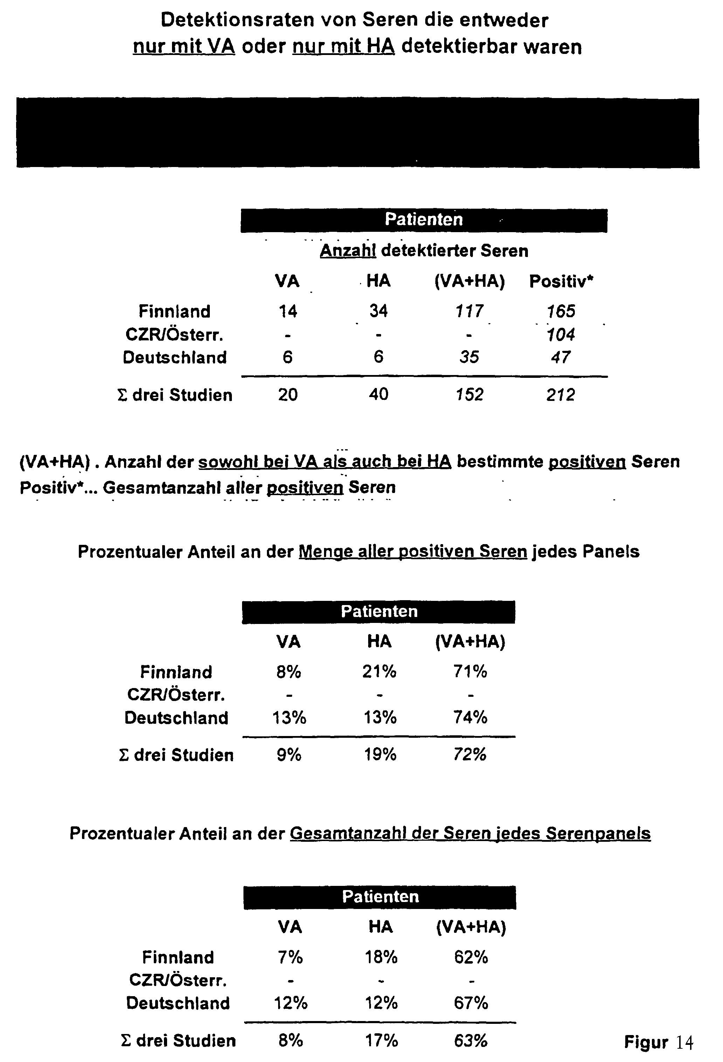

Im Zuge dieser Analysen wurde eine sehr hohe Prävalenz in der finnischen Kontrollgruppe festgestellt (die finnischen Seren stammen von den Åaland Inseln - einer stark endemischen Region). Um die Spezifitätswerte (aus den Ergebnissen der Kontrollgruppe berechnet) der angewandten Kriterien nicht zu verfälschen, wurden ausschliesslich die Ergebnisse der CZR/Österreich- und der Deutschland-Studie als Datengrundlage angenommen. Bei der Berechnung der Sensitivität konnten die Serenpanels aller drei Studien als Datengrundlage herangezogen werden. Lediglich der Sensitivitätswert der Horizontalanalyse bei der CZR/Österreich-Studie musste ausgespart werden, da in dieser Studie nur jene Seren über die HA bewertet wurden, die über die Vertikalanalyse negativ befundet worden waren (s.o.).
Führt man eine Kombination der beiden Analyseformen durch, erhält man, gemessen an den vorliegenden Serenpanels, sowohl für die Spezifität als auch für die Sensitivität jeweils Werte von 85%. Die Detektionsrate vom Lyme Borreliose serokonvertierten Seren, die entweder mit der VA oder HA detektierbar waren, sind in Fig. 14 angeführt. Figur 14 zeigt einen direkten Vergleich der Effizienz von VA und HA. Dabei zeigt sich, dass im Mittel etwa 72% der als "positiv" bewerteten Seren von jeweils beiden Analyseformen erkannt wurden. Darüber hinaus wurden 19% der Seren nur von der HA und 9% der Seren nur von der VA detektiert. Anders formuliert wären mit der HA allein nur 91%, mit der VA allein nur 81% der Seren (immer bezogen auf 100% durch die kombinierte Anwendung) detektierbar.

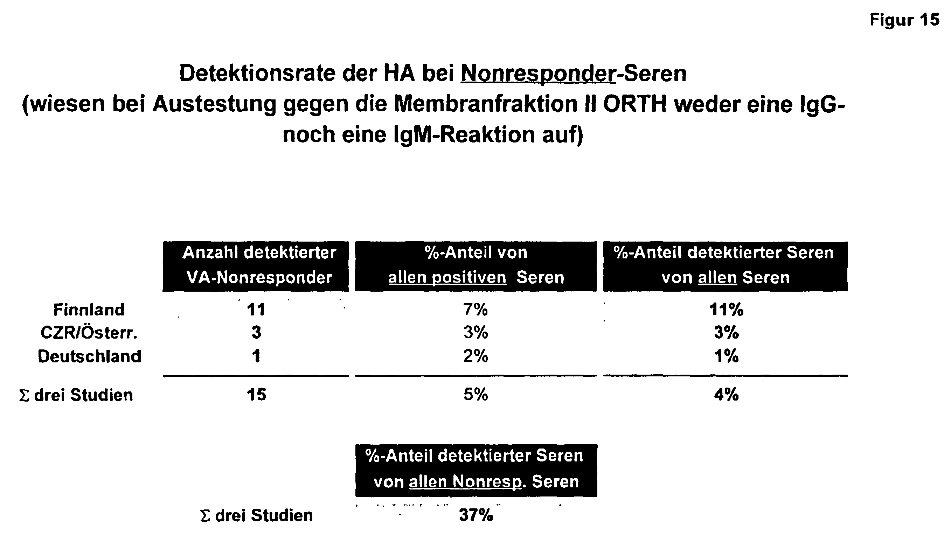

Fig. 15 zeigt ein Spezifikum der HA, nämlich auch solche Seren als positiv bewerten zu können, die keine einzige Reaktion mit Antigenen der Membranfraktion II des Stammes ORTH ergaben. Diese Tabelle zeigt, dass etwa 37% all dieser Seren, über eine HA dennoch als "positiv" befundet werden konnten. Bezogen auf die Gesamtzahl aller positiven Seren ergibt dies einen Anteil von 5%.

Begründet wird dies durch die Tatsache, dass bei einem solcherart durchgeführten serologischen Test nur heterologe Reaktionen gemessen werden können. In der Frühphase einer Infektion werden jedoch sehr häufig Antikörper gegen das OspC Antigen. Da das Antigen OspC jedoch in mehrere serologische Gruppen unterscheidbar ist und bei der VA nur eine einzige Variante enthalten ist (jene, welche dem Stamm ORTH angehört), ist die Sensitivität der HA bei diesen "Non-Responder Seren" höher.

### Beispiel 4:

### Bestimmung der Serokonversion bei Frühphasepatienten

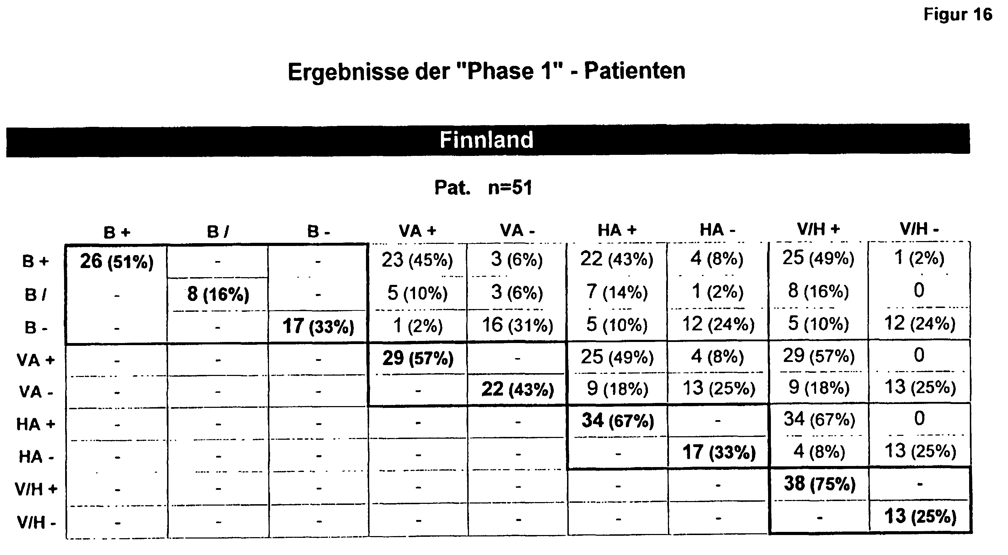

Eine Vertikal-und Horizontalanalyse wurde mit Lyme Borreliose Frühphase-Patienten der Finnland-Studie durchgeführt, da bei dieser Gruppe der Anteil an Frühphase-Seren am häufigsten war. Fig. 16 zeigt einen Vergleich der verschiedenen Analyseformen bei Frühphasepatienten. In der kombinierten Anwendung der VA und der HA wurden um 50% mehr Seren als positiv erkannt als vergleichsweise beim Bandenzahlkriterium. Insgesamt konnten bei der kombinierten Anwendung 75% der Frühphaseseren als "positiv" klassifiziert werden. Elf der 22 Seren ergaben bei der Vertikalanalyse keine einzige Reaktion und wurden als "negativ" bewertet. Drei dieser 11 Seren konnten über die HA als "positiv" befundet werden.

## Patentansprüche

1. Diagnostisches Mittel, **dadurch gekennzeichnet, daß** es als Reagenskomponente OspC Antigene abgeleitet von sämtlichen der Borrelien-RFLP-Typen 1, 2, 5, 7, 8, 9, 10, 12, 13, 14, 15, 16, 17, 19, 20, 21, 22, 23, 25 und 34 umfasst.

2. Diagnostisches Mittel nach Anspruch 1, **dadurch gekennzeichnet, daß** es eine Membranfraktion umfaßt, welche von den Borrelien-RFLP-Typen 1, 2, 5, 7, 8, 9, 10, 12, 13, 14, 15, 16, 17, 19, 20, 21, 22, 23, 25 und 34 abgeleitet ist.

3. Diagnostisches Mittel nach Anspruch 1, **dadurch gekennzeichnet, daß** die Reagenskomponenten rekombinante OspC Antigene sind.

4. Diagnostisches Mittel nach Anspruch 1, **dadurch gekennzeichnet, daß** die Reagenskomponenten gereinigte OspC-Antigene von Borrelien sind.

5. Diagnostisches Mittel nach einem der Ansprüche 1 bis 4, **dadurch gekennzeichnet, daß** die Antigene an einen festen Träger gebunden sind.

6. Diagnostische Mittel nach einem der Ansprüche 1 bis 5, **dadurch gekennzeichnet, daß** der feste Träger ausgewählt ist aus Platten, Streifen, Membranen, Papier, Film oder Perlen.

7. Verwendung eines diagnostischen Mittels nach einem der Ansprüche 1 bis 6 zum Nachweis von Lyme Borreliose und/oder zum Bestimmen des Serotyps des Borrelien-Erregers.

8. Testkit zum Nachweis von Lyme Borreliose und/oder Bestimmung des Serotyps des Borrelien-Erregers enthaltend
(i) ein diagnostisches Mittel gemäß einem der Ansprüche 1 bis 7
(ii) Substanzen zur Durchführung einer Detektion eines Antigen/Antikörperkomplexes
(iii) ein Auswertungssystem zum Erstellen einer Serodiagnose und Instruktionen.

9. Kombinationstestkit enthaltend ein
(i) ein diagnostisches Mittel gemäß einem der Ansprüche 1 bis 7
(ii) ein diagnostisches Mittel enthaltend als Reagenskomponenten mindestens 4 der von einer Membranfraktion II von *B. afzelii* abgeleitete Antigene P870, P412, P402, P365, P315, P310, P280, P251, P230 und P180 mit einem Molekulargewicht von 87,0 kD, 41,2 kD, 40,2 kD, 36, 5 kD, 31, 5 kD, 31, 0 kD, 28, 0 kD, 25, 0 kD, 23,0 kD und 18,0 kD ermittelt im SDS-PAGE mit Sammelgel: 3% Acrylamid/2,6% Bis-Acrylamid Trenngel: 11% Acrylamid/1,8 % Bis-Acrylamid, Lauflänge des Gels: 125 mm,
(iii) Substanzen zur Durchführung einer Detektion eines Antigen/Antikörperkomplexes
(iv) ein Auswertungssystem zum Erstellen einer Serodiagnose und Instruktionen, wobei die Membranfraktion II in (ii) erhältlich ist durch Aufschließen von Borrelien, Filtrieren des Lysats und Zentrifugation desselben zur Gewinnung eines Zellpellets enthaltend die Membranfraktion II.

10. Immun-Assay zum Nachweis eines Antikörpers gegen *B. burgdorferi* umfassend die Schritte
(i) Bereitstellen eines diagnostischen Mittels enthaltend als Reagenskomponenten mindestens 4 der von einer Membranfraktion II von *B. afzelii* abgeleitete Antigene P870, P412, P402, P365, P315, P310, P280, P251, P230 und P180 mit einem Molekulargewicht von 87,0 kD, 41,2 kD, 40,2 kD, 36,5 kD, 31,5 kD, 31,0 kD, 28,0 kD, 25,0 kD, 23,0 kD und 18,0 kD ermittelt im SDS-PAGE mit Sammelgel : 3% Acrylamid/2,6% Bis-Acrylamid, Trenngel: 11% Acrylamid/1,8% Bis-Acrylamid, Lauflänge des Gels: 125 mm;
(ii) Inkubieren einer Probe, die möglicherweise anti-*B. burgdorferi* Antikörper enthält, mit dem diagnostischen Mittel unter Bedingungen, die eine Ausbildung eines Antikörper/Antigen-Komplexes erlauben und
(iii) Bestimmen der Anwesenheit von anti-*B. burgdorferi* Antikörpern in der Probe durch Detektion eines Antigen/Antikörper-Komplexes, insbesondere der Komplexbildung eines Antikörpers mit mindestens einem Antigen ausgewählt aus der Gruppe von P870, P412, P402, P365, P315, P310, P280, P251, P230 und P180, wobei die Membranfraktion II in (i) erhältlich ist durch Aufschließen von Borrelien, Filtrieren des Lysats und Zentrifugation desselben zur Gewinnung eines Zellpellets enthaltend die Membranfraktion II.

11. Immun-Assay nach Anspruch 10, **dadurch gekennzeichnet, daß** die Reagenskomponenten rekombinante Antigene sind.

12. Immun-Assay nach Anspruch 10, **dadurch gekennzeichnet, daß** die Reagenskomponenten gereinigte Antigene von Borrelien sind.

13. Immun-Assay nach einem der Ansprüche 10 bis 12, **dadurch gekennzeichnet, daß** die Antigene an einen festen Träger gebunden sind.

14. Immun-Assay nach einem der Ansprüche 10 bis 13, **dadurch gekennzeichnet, daß** der feste Träger ausgewählt ist aus Platten, Streifen, Membranen, Papier, Film oder Perlen.

15. Immun-Assay nach Anspruch 10, **dadurch gekennzeichnet, daß** die biologische Probe ausgewählt ist aus tierischen oder menschlichen Flüßigkeiten, wie Blut, Plasma, Serum, Urin, Liquor und Synovialflüßigkeit.

16. Immun-Assay zum Nachweis von anti-B. burgdorferi-Antikörpern und/oder Bestimmung des Serotyps des Borrelien-Erregers umfassend die Schritte
(i) Bereitstellen eines diagnostischen Mittels nach einem der Ansprüche 1 bis 6
(ii) Inkubieren der Probe, die möglicherweise anti-*B. burgdorferi* Antikörper enthält, mit dem diagnostischen Mittel und
(iii) Bestimmen der Anwesenheit von anti-*B. burgdorferi* Antikörpern in der Probe durch Detektion der Bildung eines Antigen/Antikörper-Komplexes, insbesondere der Komplexbildung eines Antikörpers mit einem OspC-Antigen.

17. Verfahren zur Serodiagnose von Lyme Borreliose umfassend die Schritte
(i) Bereitstellen eines diagnostischen Mittels enthaltend als Reagenskomponenten mindestens 4 der von einer Membranfraktion II von *B. afzelii* abgeleitete Antigene P870, P412, P402, P365, P315, P310, P280, P251, P230 und P180 mit einem Molekulargewicht von 87,0 kD, 41,2 kD, 40,2 kD, 36,5 kD, 31,5 kD, 31,0 kD, 28,0 kD, 25,0 kD, 23,0 kD und 18,0 kD ermittelt im SDS-PAGE mit Sammelgel: 3% Acrylamid/2,6% Bis-Acrylamid, Trenngel: 11% Acrylamid/1,8% Bis-Acrylamid, Lauflänge des Gels 125 mm,
(ii) Inkubieren einer biologischen Probe, die möglicherweise anti-*B. burgdorferi* Antikörper enthält, mit dem diagnostischen Mittel und
(iii) Bestimmen der Anwesenheit von anti-*B. burgdorferi* Antikörpern in der Probe durch Detektion eines Antigen/Antikörper-Komplexes,
wobei insbesondere die Bildung eines Komplexes zwischen einem Antikörpers und einem Antigen ausgewählt aus der Gruppe von P870, P412, P402, P365, P315, P310, P280, P251, P230 und P180 indikativ für eine Lyme Borreliose ist, wobei die Membranfraktion II in (i) erhältlich ist durch Aufschließen von Borrelien, Filtrieren des Lysats und Zentrifugation desselben zur Gewinnung eines Zellpellets enthaltend die Membranfraktion II.

18. Verfahren zur Serodiagnose von Lyme Borreliose umfassend die Schritte
(i) Bereitstellen eines diagnostischen Mittels nach einem der Ansprüche 1 bis 7
(ii) Inkubieren einer biologischen Probe, die möglicherweise anti-*B*. *burgdorferi* Antikörper enthält, mit dem diagnostischen Mittel und
(iii) Bestimmen der Anwesenheit von anti-*B*. *burgdorferi* Antikörpern in der Probe durch Detektion eines Antigen/Antikörper-Komplexes,
wobei insbesondere die Bildung eines Komplexes zwischen einem Antikörpers und einem Antigen ausgewählt aus der Gruppe OspC Antigene abgeleitet von den Borrelien-RFLP-Typen 1, 2, 5, 7, 8, 9, 10, 12, 13, 14, 15, 16, 17, 19, 20, 21, 22, 23, 25 und 34 indikativ für eine Lyme Borreliose ist.

19. Verfahren nach Anspruch 17 oder 18, **dadurch gekennzeichnet, daß** die Probe in eine Gruppe eingeteilt wird, wobei die 1. Gruppe eine Reaktion zwischen einem Antikörper und mindestens einer Antigen-Reagenskomponente zeigt und damit in bezug auf Lyme Borreliose als serokonvertiert gilt und die 2. Gruppe keine Reaktion mit einem der Antigene aufweist.

20. Verfahren nach Anspruch 19, **dadurch gekennzeichnet, daß** bei der 1. Gruppe eine weiteren Bestimmung des Serotyps des Borrelien-Erregers gemäß einem Immun-Assay nach Anspruch 16 erfolgt.

21. Verfahren nach Anspruch 19, **dadurch kennzeichnet, daß** die Probe der 2. Gruppe einem Immun-Assay gemäß einem der Ansprüche 10 oder gemäß Anspruch 16 unterzogen wird.

22. Verfahren nach Anspruch 21, **dadurch gekennzeichnet, daß** gegebenenfalls die 2. Gruppe nach einer vorgegebenen Zeit erneut einer Serodiagnose gemäß einem Verfahren nach einem der Ansprüche 17 bis 21 unterzogen wird.

23. Monoklonale Antikörper gegen immunologisch aktive Antigene P870, P412, P402, P365, P315, P310, P280, P251, P230 und P180 einer Membranfraktion II von *B. afzelii* mit einem Molekulargewicht von 87,0 kD, 41,2 kD, 40,2 kD, 36,5 kD, 31,5 kD, 31,0 kD, 28,0 kD, 25,0 kD, 23,0 kD und 18,0 kD ermittelt im SDS-PAGE mit Sammelgel: 3% Acrylamid/2,6% Bis-Acrylamid, Trenngel: 11% Acrylamid/1,8% Bis-Acrylamid, Lauflänge des Gels: 125 mm wobei die Membranfraktion II erhältlich ist durch Aufschließen von Borrelien, Filtrieren des Lysats und Zentrifugation desselben zur Gewinnung eines Zellpellets enthaltend die Membranfraktion II.

24. Verwendung der monoklonalen Antikörper nach Anspruch 23 in einem Immunenzymtest zum Nachweis von Borrelien-Antigenen in einer Probe.

25. Verwendung der monoklonalen Antikörper nach Anspruch 23 zur Reinigung von immunologisch aktiven Antigen von Borrelien.

26. Isolierte, immunologisch reaktive Antigene P870, P412, P402, P365, P315, P310, P280, P230 und P180 abgeleitet von einer Membranfraktion II von *B. afzelii* Stamm Orth mit einem Molekulargewicht von 87,0 kD, 4,12 kD, 40,2 kD, 36,5 kD, 31,5 kD, 31,0 kD, 28,0 kD, 25,0 kD, 23,0 kD und 18,0 kD ermittelt im SDS-PAGE mit Sammelgel : 3% Acrylamid/2,6% Bis-Acrylamid, Trenngel: 11% Acrylamid/1,8% Bis-Acrylamid, Lauflänge des Gels: 125 mm, wobei die Membranfraktion II erhältlich ist durch Aufschließen von Borrelien, Filtrieren des Lysats und Zentrifugation desselber zur Gewinnung eines Zellpellets enthaltend die Membranfraktion II.

## Claims

1. Diagnostic agent, **characterised in that** it comprises as reagent component OspC antigens derived from all of the Borrelia RFLP types 1, 2, 5, 7, 8, 9, 10, 12, 13, 14, 15, 16, 17, 19, 20, 21, 22, 23, 25 and 34.

2. Diagnostic agent according to claim 1, **characterised in that** it comprises a membrane faction which is derived from the Borrelia RFLP types 1, 2, 5, 7, 8, 9, 10, 12, 13, 14, 15, 16, 17, 19, 20, 21, 22, 23, 25 and 34.

3. Diagnostic agent according to claim 1, **characterised in that** the reagent components are recombinant OspC antigens.

4. Diagnostic agent according to claim 1, **characterised in that** the reagent components are purified OspC antigens of Borrelia.

5. Diagnostic agent according to one of claims 1 to 4, **characterised in that** the antigens are bound to a solid carrier.

6. Diagnostic agent according to one of claims 1 to 5, **characterised in that** the solid carrier is selected from plates, strips, membranes, paper, film or beads.

7. Use of a diagnostic agent according to one of claims 1 to 6 for the detection of Lyme borreliosis and/or for determining the serotype of the Borrelia pathogen.

8. Test kit for the detection of Lyme borreliosis and/or determining the serotype of the Borrelia pathogen containing
(i) a diagnostic agent according to one of claims 1 to 7
(ii) substances for carrying out detection of an antigen/antibody complex
(iii) an evaluation system for establishing a serodiagnosis and instructions.

9. Combination test kit containing a
(i) a diagnostic agent according to one of claims 1 to 7
(ii) a diagnostic agent containing as reagent components at least 4 of the antigens P870, P412, P402, P365, P315, P310, P280, P251, P230 and P180 derived from a membrane fraction II of *B. afzelii* and having a molecular weight of 87.0 kD, 41.2 kD, 40.2 kD, 36.5 kD, 31.5 kD, 31.0 kD, 28.0 kD, 25.0 kD, 23.0 kD and 18.0 kD determined by SDS-PAGE with collecting gel: 3% acrylamide/2.6% bis-acrylamide, separating gel: 11 % acrylamide/1.8% bis-acrylamide, run length of the gel: 125 mm
(iii) substances for carrying out detection of an antigen/antibody complex
(iv) an evaluation system for establishing a serodiagnosis and instructions, wherein the membrane fraction II in (ii) can be obtained by digesting Borrelia, filtering the lysate and centrifuging the same to recover a cell pellet containing the membrane fraction II.

10. Immunoassay for the detection of an antibody against *B. burgdorferi* comprising the steps
(i) provision of a diagnostic agent containing as reagent components at least 4 of the antigens P870, P412, P402, P365, P315, P310, P280, P251, P230 and P180 derived from a membrane fraction II of *B. afzelii* and having a molecular weight of 87.0 kD, 41.2 kD, 40.2 kD, 36.5 kD, 31.5 kD, 31.0 kD, 28.0 kD, 25.0 kD, 23.0 kD and 18.0 kD determined by SDS-PAGE with collecting gel: 3% acrylamide/2.6% bis-acrylamide, separating gel: 11% acrylamide/1.8% bis-acrylamide, run length of the gel: 125 mm;
(ii) incubating a sample, which possibly contains anti-*B. burgdorferi* antibodies, with the diagnostic agent under conditions which permit formation of an antibody/antigen complex and
(iii) determination of the presence of anti-*B. burgdorferi* antibodies in the sample by detection of an antigen/antibody complex, in particular complex formation of an antibody with at least one antigen selected from the group of P870, P412, P402, P365, P315, P310, P280, P251, P230 and P180, wherein the membrane fraction II in (i) can be obtained by digesting Borrelia, filtering the lysate and centrifuging the same to recover a cell pellet containing the membrane fraction II.

11. Immunoassay according to claim 10, **characterised in that** the reagent components are recombinant antigens.

12. Immunoassay according to claim 10, **characterised in that** the reagent components are purified antigens of Borrelia.

13. Immunoassay according to one of claims 10 to 12, **characterised in that** the antigens are bound to a solid carrier.

14. Immunoassay according to one of claims 10 to 13, **characterised in that** the solid carrier is selected from plates, strips, membranes, paper, film or beads.

15. Immunoassay according to claim 10, **characterised in that** the biological sample is selected from animal or human fluids, such as blood, plasma, serum, urine, liquor and synovial fluid.

16. Immunoassay for the detection of anti-B. burgdorferi antibodies and/or determining the serotype of the Borrelia pathogen comprising the steps
(i) provision of a diagnostic agent according to one of claims 1 to 6
(ii) incubating the sample, which possibly contains anti-*B. burgdorferi* antibodies, with the diagnostic agent and
(iii) determination of the presence of anti-*B. burgdorferi* antibodies in the sample by detection of the formation of an antigen/antibody complex, in particular the complex formation of an antibody with an OspC antigen.

17. Process for serodiagnosis of Lyme borreliosis comprising the steps
(i) provision of a diagnostic agent containing as reagent components at least 4 of the antigens P870, P412, P402, P365, P315, P310, P280, P251, P230 and P180 derived from a membrane fraction II of *B. afzelii* and having a molecular weight of 87.0 kD, 41.2 kD, 40.2 kD, 36.5 kD, 31.5 kD, 31.0 kD, 28.0 kD, 25.0 kD, 23.0 kD and 18.0 kD determined by SDS-PAGE with collecting gel: 3% acrylamide/2.6% bis-acrylamide, separating gel: 11% acrylamide/1.8% bis-acrylamide, run length of the gel: 125 mm,
(ii) incubating a biological sample, which possibly contains anti-*B. burgdorferi* antibodies, with the diagnostic agent and
(iii) determination of the presence of anti-*B. burgdorferi* antibodies in the sample by detection of an antigen/antibody complex, wherein in particular the formation of a complex between an antibody and an antigen selected from the group of P870, P412, P402, P365, P315, P310, P280, P251, P230 and P180 is indicative of Lyme borreliosis, wherein the membrane fraction II in (i) can be obtained by digesting Borrelia, filtering the lysate and centrifuging the same to recover a cell pellet containing the membrane fraction II.

18. Process for serodiagnosis of Lyme borreliosis comprising the steps
(i) provision of a diagnostic agent according to one of claims 1 to 7
(ii) incubating a biological sample, which possibly contains anti-*B*. *burgdorferi* antibodies, with the diagnostic agent and
(iii) determination of the presence of anti-*B*. *burgdorferi* antibodies in the sample by detection of an antigen/antibody complex, wherein in particular the formation of a complex between an antibody and an antigen selected from the group OspC antigens derived from the Borrelia RFLP types 1, 2, 5, 7, 8, 9, 10, 12, 13, 14, 15, 16, 17, 19, 20, 21, 22, 23, 25 and 34 is indicative of Lyme borreliosis.

19. Process according to claim 17 or 18, **characterised in that** the sample is classified into a group, wherein the 1st group shows a reaction between an antibody and at least one antigen reagent component and hence is regarded as seroconverted with respect to Lyme borreliosis and the 2nd group has no reaction with one of the antigens.

20. Process according to claim 19, **characterised in that** in the 1st group, a further determination of the serotype of the Borrelia pathogen takes place according to an immunoassay according to claim 16.

21. Process according to claim 19, **characterised in that** the sample of the 2nd group is subjected to an immunoassay according to one of claims 10 or according to claim 16.

22. Process according to claim 21, **characterised in that** the 2nd group, after a preset time, is optionally subjected again to serodiagnosis according to a process according to one of claims 17 to 21.

23. Monoclonal antibodies for immunologically active antigens P870, P412, P402, P365, P315, P310, P280, P251, P230 and P180 of a membrane fraction II of *B. afzelii* having a molecular weight of 87.0 kD, 41.2 kD, 40.2 kD, 36.5 kD, 31.5 kD, 31.0 kD, 28.0 kD, 25.0 kD, 23.0 kD and 18.0 kD determined by SDS-PAGE with collecting gel: 3% acrylamide/2.6% bis-acrylamide, separating gel: 11% acrylamide/1.8% bis-acrylamide, run length of the gel: 125 mm, wherein the membrane fraction II can be obtained by digesting Borrelia, filtering the lysate and centrifuging the same to recover a cell pellet containing the membrane fraction II.

24. Use of the monoclonal antibodies according to claim 23 in an immunoenzyine test for the detection of Borrelia antigens in a sample.

25. Use of the monoclonal antibodies according to claim 23 for purifying immunologically active antigen of Borrelia.

26. Isolated, immunologically reactive antigens P870, P412, P402, P365, P315, P310, P280, P230 and P180 derived from a membrane fraction II of *B. afzelii* strain Orth and having a molecular weight of 87.0 kD, 4.12 kD, 40.2 kD, 36.5 kD, 31.5 kD, 31.0 kD, 28.0 kD, 25.0 kD, 23.0 kD and 18.0 kD determined by SDS-PAGE with collecting gel: 3% acrylamide/2.6% bis-acrylamide, separating gel: 11% acrylamide/1.8% bis-acrylamide, run length of the gel: 125 mm, wherein the membrane fraction II can be obtained by digesting Borrelia, filtering the lysate and centrifuging the same to recover a cell pellet containing the membrane fraction II.

## Revendications

1. Agent diagnostique, **caractérisé en ce qu'**il comprend, à titre de composants réactifs, des antigènes OspC dérivés de l'ensemble des types borrelia-RFLP 1, 2, 3, 5, 7, 8, 9, 10, 12, 13, 14, 15, 16, 17, 19, 20, 21, 22, 23, 25 et 34.

2. Agent diagnostique selon la revendication 1, **caractérisé en ce qu'**il comprend une fraction membrane dérivée des types borrelia-RFLP 1, 2, 3, 5, 7, 8, 9, 10, 12, 13, 14, 15, 16, 17, 19, 20, 21, 22, 23, 25 et 34.

3. Agent diagnostique selon la revendication 1, caractérisé en ce les composants réactifs sont des antigènes OspC recombinants.

4. Agent diagnostique selon la revendication 1, caractérisé en ce les composants réactifs sont des antigènes OspC épurés de borrelia.

5. Agent diagnostique selon une des revendications 1 à 4, caractérisé en ce les antigènes sont liés à un support fixe.

6. Agent diagnostique selon une des revendications 1 à 5, **caractérisé en ce que** le support fixe est sélectionné parmi des plaques, des bandes, des membranes, du papier, un film ou des perles.

7. Utilisation d'un agent diagnostic selon une des revendications 1 à 6, pour détecter la borréliose de Lyme et/ou pour déterminer le sérotype de l'excitateur borrelia

8. Kit de tests pour détecter la borréliose de Lyme et/ou pour la détermination du sérotype de l'excitateur borrelia, contenant
(i) un agent diagnostic selon l'une des revendications 1 à 7
(ii) des substances pour effectuer une détection d'un complexe antigène/anticorps
(iii) un système d'évaluation pour établir un sérodiagnostic et des instructions.

9. Kit de tests de combinaison contenant :
(i) un agent diagnostic selon l'une des revendications 1 à 7
(ii) un agent diagnostic contenant, en tant que composant réactif, au moins 4 des antigènes P870, P412, P402, P365, P315, P310, P280, P251, P230 et P180, dérivés d'une fraction membrane II de *B. afzelii* avec un poids moléculaire de 87,0 kD, 41,2 kD, 40,2 kD, 36,5 kD, 31,5 kD, 31,0 kD, 28,0 kD, 25,0 kD, 23,0 kD, et 18,0 kD, déterminé dans SDS-page, avec comme gel collecteur : 3% d'acrylamide/2,6% de bis-acrylamide, comme gel séparateur : 11% d'acrylamide /1,8% de bis-acrylamide, longueur de passage du gel : 125 mm
(iii) des substances pour effectuer une détection d'un complexe antigène/anticorps,
(iv) un système d'évaluation pour établir un sérodiagnostic et des instructions, la fraction membrane II étant disponible dans (ii) par décomposition de borrelia, filtrage du lysat et centrifugation de celui-ci pour obtenir une boulette cellulaire contenant la fraction membrane II.

10. Essai immunologique pour la détection des anticorps anti-*B*. *burgdorferi* comprenant les étapes de :
(i) fourniture d'un agent diagnostique contenant, en tant que composants réactifs, au moins 4 des antigènes P870, P412, P402, P365, P315, P310, P280, P251, P230 et P180, dérivés d'une fraction membrane II de *B. afzelii*, avec un poids moléculaire de 87,0 kD, 41,2 kD, 40,2 kD, 36,5 kD, 31,5 kD, 31,0 kD, 28,0 kD, 25,0 kD, 23,0 kD, et 18,0 kD, déterminé dans SDS-page, avec comme gel collecteur : 3% d'acrylamide/2,6% de bis-acrylamide, comme gel séparateur : 11% d'acrylamide /1,8% de bis-acrylamide, longueur de passage du gel : 125 mm
(ii) incubation d'un échantillon, contenant éventuellement des anticorps anti-*B. burgdorferi*, avec l'agent diagnostique, dans des conditions permettant la formation d'un complexe anticorps/antigène, et
(iii)détermination de la présente des anticorps anti-*B*. *burgdorferi* dans l'échantillon, par détection d'un complexe antigène/anticorps, en particulier de la formation d'un complexe d'un anticorps avec au moins un antigène sélectionné dans le groupe de P870, P412, P402, P365, P315, P310, P280, P251, P230 et P180, la fraction membrane II étant disponible dans (i) et par décomposition de Borrelias, filtration du lysat et centrifugation de celui-ci pour obtenir une boulette cellulaire contenant la fraction membrane II.

11. Essai immunologique selon la revendication 10, **caractérisé en ce que** les composants réactifs sont des antigènes recombinants.

12. Essai immunologique selon la revendication 10, **caractérisé en ce que** les composants réactifs sont des antigènes épurés de Borrelia.

13. Essai immunologique selon l'une des revendications 10 à 12, **caractérisé en ce que** les antigènes sont liés à un support fixe.

14. Essai immunologique selon l'une des revendications 10 à 13, **caractérisé en ce que** le support fixe est sélectionné parmi des plaques, des bandes, des membranes, du papier, un film ou des perles.

15. Essai immunologique selon la revendication 10, **caractérisé en ce que** l'échantillon biologique est sélectionné parmi des liquides animaux ou humains, tels que du sang, du plasma, du sérum, de l'urine, un liquide organique et un liquide synovial.

16. Essai immunologique pour la détection des anticorps anti-*B. burgdorferi* et/ou la détermination du sérotype de l'excitateur Borrelia, comprenant les étapes consistant à :
(i) fournir un agent diagnostique selon l'une des revendications 1 à 6
(ii) faire incuber l'échantillon qui contient éventuellement des anticorps anti-*B. burgdorferi*, avec l'agent diagnostique, et
(iii) déterminer la présence d'anticorps anti-*B. burgdorferi* dans l'échantillon, par détection de la formation d'un complexe antigène/anticorps, en particulier de la formation d'un complexe d'un anticorps avec un antigène OspC.

17. Procédé de sérodiagnostic de la borréliose de Lyme comprenant les étapes de :
(i) fourniture d'un agent diagnostique contenant, en tant que composants réactifs, au moins 4 des antigènes P870, P412, P402, P365, P315, P310, P280, P251, P230 et P180, dérivés d'une fraction membrane II de *B. afzelii*, avec un poids moléculaire de 87,0 kD, 41,2 kD, 40,2 kD, 36,5 kD, 31,5 kD, 31,0 kD, 28,0 kD, 25,0 kD, 23,0 kD, et 18,0 kD, déterminé dans SDS-page, avec comme gel collecteur : 3% d'acrylamide/2,6% de bis-acrylamide, comme gel séparateur : 11% d'acrylamide /1,8% de bis-acrylamide, longueur de passage du gel : 125 mm
(ii) incubation d'un échantillon biologique, contenant éventuellement des anticorps anti-*B*. *burgdorferi*, avec l'agent diagnostique, et
(iii) détermination de la présence d'anticorps anti-*B*. *burgdorferi* dans l'échantillon, par détection d'un complexe antigène/anticorps, sachant qu'en particulier la formation d'un complexe entre un anticorps et un antigène, sélectionné dans le groupe de P870, P412, P402, P365, P315, P310, P280, P251, P230 et P180 est indicative d'une borréliose de Lyme, la fraction membrane II dans (i) étant obtenue par décomposition de Borrelias, filtration du Lysat et centrifugation de celui-ci pour obtenir une boulette cellulaire contenant la fraction membrane II.

18. Procédé de sérodiagnostic de la borréliose de Lyme comprenant les étapes consistant à :
(i) fournir un agent diagnostique selon l'une des revendications 1 à 7
(ii) faire incuber un échantillon qui contient éventuellement des anticorps anti-*B. burgdorferi*, avec l'agent diagnostique, et
(iii) déterminer la présence d'anticorps anti-*B. burgdorferi* dans l'échantillon, par détection de la formation d'un complexe antigène/anticorps,où , en particulier, la formation d'un complexe entre un anticorps et un antigène sélectionné dans le groupe des antigènes OspC, dérivé des types borrelia-RFLP 1, 2, 3, 5, 7, 8, 9, 10, 12, 13, 14, 15, 16, 17, 19, 20, 21, 22, 23, 25 et 34 estindicative d'une borréliose de Lyme.

19. Procédé selon la revendication 17 ou 18, **caractérisé en ce que** l'échantillon est classé dans un groupe, le groupe 1 présentant une réaction un anticorps et au moins un composant réactif antigène et ainsi valant comme étant séroconverti en référence à la borréliose de Lyme, et le groupe 2 ne présentant aucune réaction avec l'un des antigènes.

20. Procédé selon la revendication 19, **caractérisé en ce que**, dans le cas du groupe 1, une détermination supplémentaire du sérotype de l'excitateur Borrelia est effectuée selon un essai immunologique, selon la revendication 16.

21. Procédé selon la revendication 19, **caractérisé en ce que** l'échantillon du groupe 2 est soumis à un essai immunologique selon l'une des revendications 10 ou selon la revendication 16.

22. Procédé selon la revendication 21, **caractérisé en ce que**, le cas échéant le groupe 2, après expiration d'une durée prédéterminée est de nouveau soumis à un sérodiagnostic suivant un procédé selon l'une des revendications 17 à 21.

23. Anticorps monoclonal contre les antigènes immunologiquement actifs P870, P412, P402, P365, P315, P310, P280, P251, P230 et P180 d'une fraction membrane II de *B. afzelii* avec un poids moléculaire de 87,0 kD, 41,2 kD, 40,2 kD, 36,5 kD, 31,5 kD, 31,0 kD, 28,0 kD, 25,0 kD, 23,0 kD, et 18,0 kD, déterminé dans SDS-page, avec comme gel collecteur : 3% d'acrylamide/2,6% de bis-acrylamide, comme gel séparateur : 11% d'acrylamide /1,8% de bis-acrylamide, longueur de passage du gel : 125 mm, la fraction membrane II dans (i) étant obtenue par décomposition de Borrelias, filtration du Lysat et centrifugation de celui-ci pour obtenir une boulette cellulaire contenant la fraction membrane II.

24. Utilisation des anticorps monoclonaux selon la revendication 23, dans un test immuno-enzymatique pour la détection des antigènes Borrelia dans un échantillon.

25. Utilisation des anticorps monoclonaux selon la revendication 23, pour l'épuration d'antigène immunologiquement actif de Borrelia.

26. Antigènes isolés; immunologiquement actifs P870, P412, P402, P365, P315, P310, P280, P251, P230, et P180, dérivés d'une fraction membrane II de *B. afzelii*, souche Orth avec un poids moléculaire de 87,0 kD, 41,2 kD, 40,2 kD, 36,5 kD, 31,5 kD, 31,0 kD, 28,0 kD, 25,0 kD, 23,0 kD et 18,0 kD, déterminé dans SDS-page, avec comme gel collecteur : 3% d'acrylamide/2,6% de bis-acrylamide, comme gel séparateur : 11% d'acrylamide /1,8% de bis-acrylamide, longueur de passage du gel : 125 mm, la fraction membrane II étant disponible par décomposition de Borrelias, filtration du lysat et centrifugation de celui-ci pour obtenir une boulette cellulaire contenant la fraction membrane II.
